(19) Europäisches Patentamt
European Patent Office
Office européen des brevets

(11) **EP 4 209 218 A1**

(12) **EUROPEAN PATENT APPLICATION**
published in accordance with Art. 153(4) EPC

(43) Date of publication:
**12.07.2023 Bulletin 2023/28**

(21) Application number: **21863678.5**

(22) Date of filing: **03.09.2021**

(51) International Patent Classification (IPC):
***A61K 31/5377*** *(2006.01)* ***A61P 13/12*** *(2006.01)*
***A61P 9/12*** *(2006.01)* ***A61P 3/10*** *(2006.01)*
***A61P 9/04*** *(2006.01)* ***A61K 9/20*** *(2006.01)*
***A61K 9/48*** *(2006.01)* ***A61K 9/08*** *(2006.01)*
***A61K 9/19*** *(2006.01)* ***A61K 9/06*** *(2006.01)*
***A61K 9/02*** *(2006.01)* ***A61K 9/12*** *(2006.01)*

(52) Cooperative Patent Classification (CPC):
**A61K 9/02; A61K 9/06; A61K 9/08; A61K 9/12; A61K 9/19; A61K 9/20; A61K 9/48; A61K 31/5377; A61P 3/00; A61P 3/10; A61P 9/04; A61P 9/12; A61P 13/12; C07C 59/245; C07D 471/04**

(86) International application number:
**PCT/CN2021/116338**

(87) International publication number:
**WO 2022/048614 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.09.2020 CN 202010921155**

(71) Applicant: **Shanghai Pharmaceuticals Holding Co., Ltd.**
**Shanghai 201203 (CN)**

(72) Inventors:
- **XIA, Guangxin**
  **Shanghai 201203 (CN)**
- **SU, Wei**
  **Shanghai 201203 (CN)**
- **WANG, Xuesong**
  **Shanghai 201203 (CN)**
- **KE, Ying**
  **Shanghai 201203 (CN)**

(74) Representative: **Kador & Partner Part mbB**
**Corneliusstraße 15**
**80469 München (DE)**

# (54) APPLICATION OF NITROGEN-CONTAINING SATURATED HETEROCYCLIC COMPOUND

(57) The present invention provides a use of a nitrogen-containing saturated heterocyclic compound. Specifically, the present invention provides a use of a nitrogen-containing saturated heterocyclic compound represented by the following formula (I) or a pharmaceutically acceptable salt thereof in the preparation of a medicine for the treatment and/or prevention of chronic kidney disease:

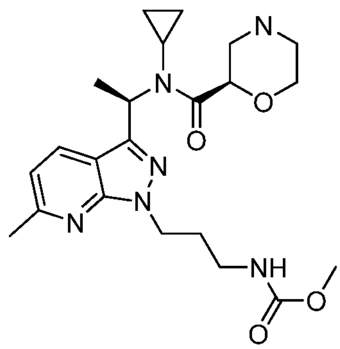

(I)

EP 4 209 218 A1

## Description

### Technical field

The present invention relates to a use of a nitrogen-containing saturated heterocyclic compound or a pharmaceutically acceptable salt thereof in the preparation of a medicine for the treatment and/or prevention of chronic kidney disease.

### Background of the Art

Chronic kidney disease (CKD) has the characteristics of high prevalence, low awareness, poor prognosis and high medical costs, which is another disease that seriously endangers human health after cardiovascular and cerebrovascular diseases, diabetes and malignant tumors. In recent years, the prevalence of CKD has increased year by year, and the global prevalence rate of the general population has reached 14.3%, the cross-sectional epidemiological study in China shows that the prevalence of CKD in people over 18 years old is 10.8%. Chronic kidney disease (CKD) is a progressive disease. If the disease is not treated in a timely and effective manner, the condition worsens and progresses, as the course of the disease progresses, patients with chronic kidney disease will develop renal failure. In such a case, without artificial dialysis or kidney transplantation, survival will become difficult. There is currently no particularly effective method for the treatment of chronic kidney disease, therefore, it has great clinical value to actively find medicines that are effective in treating chronic kidney disease and have low toxic and side effects.

CN103562191B discloses a nitrogen-containing saturated heterocyclic compound represented by the following structural formula or a pharmaceutically acceptable salt thereof, which shows an inhibitory effect on renin and can be used to treat hypertension.

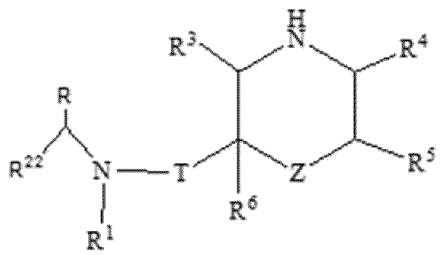

In the formula, $R^1$ represents a cycloalkane group, etc., $R^{22}$ represents a substituted aryl etc., R represents lower alkane group, etc., T represents a carbonyl group, Z represents -O- etc., $R^3$, $R^4$, $R^5$ and $R^6$ are the same or different, and represent a hydrogen atom etc.

CN106928218A discloses a salt of a novel pharmaceutically acceptable morpholine derivative (nitrogen-containing saturated heterocyclic ring), including its malate, tartrate, hydrochloride, acetate and naphthalene diphosphate, wherein, tartrate has three crystalline salt forms: crystal form A, crystal form B and dehydrate, malate, hydrochloride and acetate each have one crystalline salt form, and naphthalene diphosphate is amorphous. Compared with the known free bases of morpholine derivatives, the salts of morpholine derivatives have one or more improved properties, such as better crystalline state, which greatly improve water solubility, light stability and thermal stability, etc. The above salt of morpholine derivative or its crystal form can be used to treat and/or prevent hypertension.

### Summary of the invention

The technical problem to be solved by the present invention is to provide a use of a nitrogen-containing saturated heterocyclic compound or a pharmaceutically acceptable salt thereof in the preparation of a medicine for the treatment and/or prevention of chronic kidney disease in view of the absence of a particularly effective medicine for treating chronic kidney disease in the prior art.

The entire contents of the patent CN103562191B and the patent application CN106928218A described in the background art are hereby incorporated into this description by reference.

In one embodiment of the present invention, provided is a use of a nitrogen-containing saturated heterocyclic compound represented by the following formula I or a pharmaceutically acceptable salt thereof in the preparation of a medicine for the treatment and/or prevention of chronic kidney disease:

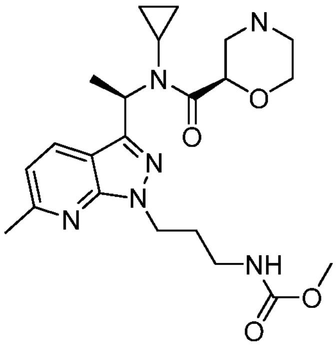

formula I

In a further embodiment of the present invention, the pharmaceutically acceptable salt is hydrochloride, sulfate, phosphate, hydrobromide, acetate, fumarate, oxalate, citrate, methanesulfonate, benzenesulfonate, p-toluenesulfonate, maleate, malate, tartrate, acetate or naphthalene disulfonate.

In a further embodiment of the present invention, the pharmaceutically acceptable salt is malate, tartrate, hydrochloride, acetate or naphthalene disulfonate.

In one embodiment of the present invention, the pharmaceutically acceptable salt is the malate of the compound of formula I, which is a compound formed by the compound of formula I and malic acid at a molar ratio of 1:1, and the structural formula is as follows:

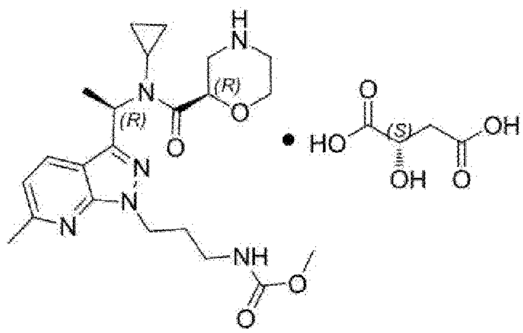

。

In one embodiment of the present invention, the malate is a crystal form, the X-ray powder diffraction pattern thereof has characteristic peaks at 2θ of 7.767°±0.2°, 13.897°±0.2°, 14.775°±0.2°, 17.098°±0.2°, 18.999°±0.2°, 20.153±0.2°, 20.960°±0.2°, 21.423°±0.2°, 26.348°±0.2° and 27.892°±0.2°. In particular, the X-ray powder diffraction pattern of the malate crystal further has characteristic peaks at 2θ of 5.598°±0.2°, 7.357°±0.2°, 10.395°±0.2°, 11.108°±0.2°, 16.037°±0.2°, 16.523°±0.2°, 19.410°±0.2°, 22.645°±0.2°, 26.630°±0.2°, 26.891°±0.2°, 27.380°±0.2°, 31.056° ±0.2°, 33.306°±0.2°, 33.775°±0.2° and 39.231°±0.2°. More specifically, the malate crystal of the compound of formula I has an X-ray powder diffraction pattern as shown in Fig. 1.

In one embodiment of the present invention, the chronic kidney disease includes hypertension with nephropathy, hypertension with nephropathy with abnormal glucose metabolism, chronic renal insufficiency with chronic heart failure or chronic kidney disease with abnormal glucose metabolism.

In one embodiment of the present invention, the chronic kidney disease refers to the G1, G2, G3a, G3b, G4 stages of chronic kidney disease, preferably the G2, G3a or G3b stage.

In one embodiment of the present invention, the hypertension in hypertension with nephropathy is grade 1 hypertension, grade 2 hypertension or grade 3 hypertension, preferably grade 1 hypertension or grade 2 hypertension.

In one embodiment of the present invention, the dosage forms of the medicine include tablet, capsule, intravenous injection, inhalant, aerosol, lyophilized agent, patch, gel, spray, or suppository, preferably tablet.

In one embodiment of the present invention, the medicine is unit dose.

In one embodiment of the present invention, the unit dose of the medicine contains the nitrogen-containing saturated heterocyclic compound represented by formula I or the pharmaceutically acceptable salt thereof in a range of 25 mg to 200 mg, for example, 25 mg, 50 mg, 100 mg, 150 mg, 200 mg of the nitrogen-containing saturated heterocyclic compound represented by formula I or the pharmaceutically acceptable salt thereof.

The "Kidney Disease Outcomes Quality Initiative" (KDOQI) working group under the American Kidney Foundation (NKF) formulated the definition and staging criteria for CKD in 2002.

Chronic kidney disease is defined as abnormal kidney structure or function> 3 months. The diagnostic criteria for chronic kidney disease is that any of the indicators in Table 1 below is present for ≥ 3 months.

Table 1: Diagnostic criteria for chronic kidney disease

| | |
|---|---|
| Kidney damage signs | (1) Albuminuria [AER≥30 mg/24 h; ACR≥30 mg/g(or≥3 mg/mmol)];<br>(2) Abnormal urine sediment;<br>(3) Renal tubule-associated pathological changes;<br>(4) Histological abnormality;<br>(5) Structural abnormalities detected by imaging;<br>(6) History of kidney transplantation |
| Decreased GFR | eGFR<60 mL/(min- 1.73m$^2$) |
| Note: meeting at least one item: AER: Urinary albumin excretion rate; ACR: Urinary albumin to creatinine ratio; GFR: glomerular filtration rate | |

Chronic kidney disease staging: Chronic kidney disease is divided into 5 stages according to the glomerular filtration rate (GFR), see Table 2 below.

Table 2: Glomerular Filtration Rate GFR categories - Description and range

| Staging | GFR [mL/(min•1.73m$^2$)] | Description |
|---|---|---|
| G1 | ≥90 | Normal or high |
| G2 | 60-89 | Mildly decreased |
| G3a | 45-59 | Mildly to moderately decreased |
| G3b | 30-44 | |
| G4 | 15-29 | Moderately to severely decreased |
| G5 | <15 | |
| | | Severely decreased |
| | | Kidney failure |

Definition of hypertension: systolic blood pressure (SBP) ≥140 mmHg (1 mmHg = 0.133 kPa) and/or diastolic blood pressure (DBP) ≥90 mmHg, in the case of not using antihypertensive drugs and that office blood pressure is measured 3 times on different days.

That SBP≥140 mmHg and DBP<90 mmHg is isolated systolic hypertension. When the patient has a history of hypertension and is currently using antihypertensive drugs, although the blood pressure is lower than 140/90 mmHg, it should still be diagnosed as hypertension.

According to the elevated blood pressure level, hypertension is further divided into Grade 1, 2 or 3. The classification and definition of blood pressure levels are shown in Table 3 below.

Table 3: Classification and definition of blood pressure levels

| Classification | Systolic blood pressure ( mmHg ) | | Diastolic blood pressure ( mmHg ) |
|---|---|---|---|
| Normal | < 120 | and | < 80 |
| Elevated | 120-139 | and/or | 80-90 |
| Hypertension | ≥140 | and/or | ≥90 |
| Grade 1 hypertension (mild) | 140-159 | and/or | 90-99 |
| Grade 2 hypertension (moderate) | 160-179 | and/or | 100-109 |
| Grade 3 hypertension (severe) | ≥180 | and/or | ≥110 |
| Isolated systolic hypertension | ≥140 | and | < 90 |

Heart failure is a clinical syndrome, defined as a group of complex clinical syndromes in which the ability of the ventricle to fill with or eject blood is impaired due to any abnormality in the structure or function of the heart. The main

clinical manifestations are dyspnea and fatigue (restricted activity tolerance), and fluid retention (pulmonary congestion and peripheral edema).

According to the left ventricular ejection fraction (LVEF), heart failure is divided into heart failure with reduced ejection fraction (HFrEF), heart failure with preserved ejection fraction, HFpEF) and heart failure with mid - range ejection fraction (HFmrEF), the definitions of the 3 types of heart failure are shown in Table 4 below:

Table 4: Classification and definition of heart failure

| Classification | Symptoms and/or signs | Left ventricular ejection fraction (%) | Others |
|---|---|---|---|
| HFrEF | Presented | < 40 | - |
| HFmrEF | Presented | 40-49 | 1. Natriuretic peptide increased [a];<br>2. Meeting at least one of the following:<br>(1) Left ventricular hypertrophy and/or left atrium enlargement;<br>(2) Abnormal diastolic function [b] |
| HFpEF | Presented | ≥50 | 1. Natriuretic peptide increased [a];<br>2. Meeting at least one of the following:<br>(1) Left ventricular hypertrophy and/or left atrium enlargement;<br>(2) Abnormal diastolic function [b] |

Notes: HFrEF, heart failure with reduced ejection fraction; HFmrEF, heart failure with median ejection fraction; HFpEF, heart failure with preserved ejection fraction; [a] B-type natriuretic peptide (BNP)>35 ng/L and/or N-terminal B-type pronatriuretic peptide (NT-proBNP)>125 ng/L; b E/e'≥13, e' average value (ventricular septum and free wall) <9 cm/s; - none

Abnormal glucose metabolism includes pre-diabetes and diabetes.

Pre-diabetes is impaired glucose regulation, which means that the blood sugar level has exceeded the normal range, but has not yet reached the diagnostic criteria for diabetes, namely, the intermediate state between normal people and diabetic patients.

Diabetes is a group of common metabolic diseases characterized by hyperglycemia, honey-urine, impaired glucose tolerance and abnormal insulin release in test.

The positive and progressive effects of the present invention are: studies on the effectiveness and safety of nitrogen-containing saturated heterocyclic compound salt administered continuously for 8 weeks to rhesus monkeys (middle-aged/elderly) with chronic renal insufficiency with chronic heart failure have shown that, the nitrogen-containing saturated heterocyclic compound or the pharmaceutically acceptable salt thereof provided by the present invention can effectively prevent or treat chronic kidney disease, the chronic kidney disease includes hypertension with nephropathy, hypertension with nephropathy with abnormal glucose metabolism, chronic renal insufficiency with chronic heart failure or chronic kidney disease with abnormal glucose metabolism. At the same time, the nitrogen-containing saturated heterocyclic compound or the pharmaceutically acceptable salt thereof provided by the present invention is very safe, and there is no adverse event related to administration.

## BRIEF DESCRIPTION OF THE DRAWINGS

Figure 1 shows the XRPD pattern of the malate of the compound of formula I used in the examples of present invention;
Figure 2 shows drug concentration-time graph of the compound of formula I in monkeys after intravenous (iv) and oral (po) administration in Example 3, in which the doses were respectively 1 mg/kg (intravenous), and 1 mg/kg, 3 mg/kg, and 9 mg/kg (oral).

## DETAILED DESCRIPTION OF EXEMPLARY EMBODIMENTS

The present invention will be further explained by way of examples below, but the present invention is not limited to the scope of the described examples. In the following examples, the experimental methods without specific conditions are selected according to conventional methods and conditions, or according to the product specification.

The malate of the compound of Formula I used in the following examples was prepared and confirmed according to the preparation method described in Example 1 in Patent Application Publication CN106928218A. Valsartan was purchased through commercial channels. The rhesus monkeys used were purchased from Sichuan Premey Xingzhi Biotechnology Co., Ltd. (Premey Primate Research Center).

Abbreviations

| Abbreviations | English full name |
|---|---|
| CKD-EPI | Chronic Kidney Epidemiology |
| GFR | glomerular filtration rate |
| eGFRcreat-cys | Estimated glomerular filtration rate (creatinine-cystatin C) |
| UACR | urine albumin creatine ratio |
| SBP | Systolic blood pressure |
| DBP | Diastolic blood pressure |
| MALB | Micro albumin |
| CR-U | Urine creatinine |
| E | Peak Velocity of Mitral Blood Flow at early diastole |
| Ea | Peak Velocity of Mitral Annulus at early diastole |
| LVEF | Left Ventricular Ejection Fraction |
| CVD | cerebrovascular disease |
| DKD | diabetic kidney disease |

**Example 1: Study on the effectiveness and safety of the malate of the compound of Formula I administered continuously for 8 weeks to rhesus monkeys with chronic renal insufficiency with chronic heart failure (middle-aged/elderly)**

**1. Research purpose**

The improvement of renal function by 8-week continuous administration of the malate of the compound of formula I, and the effective dose in which the renal function can be improved or delayed, the intensity of action and the risk of CVD events were evaluated, in order to provide basis for the dosage design of clinical trials, patient selection criteria and safety. Primary efficacy endpoints (ENDPOINT): 1. Improvement of renal function: glomerular filtration rate $eGFR_{creat\text{-}cys}$, Cystatin C (CysC), creatinine and UACR; 2. Improvement of cardiac function: analysis of changes of cardiac structure and systolic and diastolic functions before and after administration by echocardiographic indicators; 3. Safety indicators: blood pressure, blood potassium, glucose and lipid metabolism related indicators, etc.

**2. Test system**

**2.1 Rhesus monkeys with spontaneous chronic renal insufficiency**

Animal species: Macaca mulatta (Rhesus Macaque)

Level: Normal level. The pre-test quarantine was qualified, including physical examination, 2 tubercle bacillus tests, parasite, salmonella, shigella and B virus inspections.
Animal logo: A stainless steel number plate engraved with Arabic numerals was worn on the neck ring, and the chest was tattooed.
Supplier: Sichuan Primed Shines Bio-tech Co., Ltd.Production license number: SCXK (Sichuan) 2019-027.

Animal certificate number: 0016710, 0016944, 0016966.

### 2.2 Selection criteria

23 male/female animals, aged 14-24 years (equivalent to adults aged 40-70 years) were selected. The body weight thereof was 8.45-14.93 kg for males, and was 6.43 kg for one female.

They suffered from long-term abnormal glucose and lipid metabolism for more than 3 years. The abnormal glucose metabolism was fasting blood glucose (FPG)>4.8 mmol/l, vs. age-matched control group 4.1±0.3 mmol/l.

CKD-EPI rating G3a-G3b, glomerular filtration rate eGFR: 30-59 ml/min/1.73m$^2$; or moderate to severe increase in proteinuria (A2-A3): urine albumin/creatinine ratio (UACR) ≥ 15 mg/g - 350 mg/g (4-6 hour urine collection), vs. age-matched control group 3±2 mg/g.

Normal blood pressure, grade 1 hypertension or grade 2 hypertension (consistent with clinical patient standard).

HFrEF: LVEF 30%-49% (normal value: 50%-70% Simpson biplane method) or HFpEF (moderate or above damage): Ea<8 or E/Ea>10 (based on clinical diagnostic criteria).

### 2.3 Exclusion criteria

1) Chronic liver disease (including known active hepatitis) and/or screening for alanine aminotransferase (ALT) or aspartate aminotransferase (AST)> 3x upper limit of normal (LTLN).
2) Exclusion criteria related to dyslipidemia: TC> 7 mmol/l; TG> 3.5 mmol/l.
3) Any history of other diseases that may affect the evaluation of drug efficacy.

Table 5 below shows the underlying causes and characteristics of rhesus monkeys with chronic DKD renal insufficiency in baseline period-each dose group.

Table 5: The underlying causes and characteristics of rhesus monkeys with chronic DKD renal insufficiency in baseline period-each dose group.

| Group | Animal No. | Age | Body weight | Kidney function diagnosis | UACR | eGFR | CR-P | CYS C | Years of abnormal glucose and lipid metabol ism | FPG | Blood pressure (SBP/DBP) | Anemia diagnosis | RBC | HG B | $K^+$ | EF | E/Ea |
|---|---|---|---|---|---|---|---|---|---|---|---|---|---|---|---|---|---|
| | | yrs | kg | CKD-EPI rating | mg/g | (ml/min/ 1.73 $m^2$) | mg/d L | mg/ L | | mmol /L | mmHg | | $10^{12}$/ L | g/L | mmo l/L | % | / |
| Placebo group (n=4) | **6653** | 19 | 10.92 | G3a-A2 | 27.8 | 47 | 1.23 | 1.80 | ≥3 | 4.90 | 103/53 | Normal | 5.23 | 121 | 4.70 | 68.36 | 11.4 3 |
| | **231** | 18 | 10.19 | G3a-A2 | 14.5 | 58 | 1.19 | 1.42 | ≥3 | 5.79 | 134/63 | Normal | 5.57 | 135 | 5.37 | 54.05 | 10.8 7 |
| | **1029** | 21 | 12.71 | G3a | 10.5 | 53 | 1.00 | 1.71 | ≥3 | 5.35 | 162/72 | Normal | 5.69 | 143 | 3.92 | 50.79 | 10.6 3 |
| | **4713** | 21 | 11.88 | G3a | 8.5 | 47 | 1.42 | 1.55 | ≥3 | 4.86 | 143/65 | Normal | 6.09 | 140 | 4.38 | 58.81 | 26.1 6 |
| | **Mean±SD** | **20± 2** | **11.43±1. 10** | / | **15.3 ±8.7** | **51±5** | **1.21 ±0.17** | **1.62 ±0.1 7** | / | **5.23 ±0.44** | **135±24/63± 8** | / | **5.65 ±0.35** | **135 ±10** | **4.59 ±0.61** | **58.00 ±7.65** | **14.7 7 ±7.6 0** |
| Valsartan group (n=3) | **2091** | 18 | 9.35 | G3a-A2 | 37.3 | 55 | 1.28 | 1.44 | ≥3 | 7.77 | 131/67 | Normal | 6.79 | 153 | 4.63 | 64.02 | 8.40 |
| | **4861** | 17 | 11.23 | G3a-A2 | 63.6 | 50 | 1.31 | 1.62 | ≥3 | 5.75 | 125/60 | Normal | 6.77 | 152 | 4.27 | 58.75 | 13.8 0 |
| | **6645** | 23 | 9.97 | G3b-A2 | 15.0 | 40 | 1.50 | 1.76 | ≥3 | 6.06 | 148/82 | Normal | 6.09 | 137 | 5.07 | 59.26 | 18.3 5 |
| | **Mean±SD** | **19 ±3** | **10.18 ±0.96** | / | **38.7 ±24.3** | **48±7** | **1.36 ±0.12** | **1.61 ±0.1 6** | / | **6.53 ±1.09** | **135±12/ 70±11** | / | **6.55 ±0.40** | **147 ±9** | **4.66 ±0.40** | **60.68 ±2.91** | **13.5 2 ±4.9 8** |
| The group of malate of compound of Formula I 5mg/kg (n=6) | **144** | 18 | 6.43 | G3b-A2 | 118.5 | 55 | 0.90 | 1.49 | ≥3 | 5.46 | 132/63 | Normal | 6.41 | 162 | 5.43 | 63.71 | 7.09 |
| | **5073** | 14 | 9.75 | G3a | 10.5 | 48 | 1.53 | 1.63 | ≥3 | 5.32 | 159/105 | Normal | 5.92 | 143 | 4.74 | 42.11 | 11.4 6 |
| | **6651** | 24 | 8.45 | G3a-A2 | 25.4 | 51 | 1.10 | 1.61 | ≥3 | 4.23 | 127/74 | Normal | 5.64 | 139 | 4.03 | 71.95 | 9.21 |
| | **257** | 21 | 9.45 | G3b-A2 | 279.3 | 41 | 0.99 | 2.52 | ≥3 | 4.59 | 180/97 | Normal | 5.50 | 121 | 5.20 | / | / |
| | **287** | 15 | 12.87 | G3a | 2.4 | 53 | 1.43 | 1.47 | ≥3 | 6.08 | 154/70 | Normal | 5.74 | 134 | 4.75 | 49.98 | 5.91 |
| | **4721** | 17 | 10.38 | G3b-A2 | 125.8 | 43 | 1.79 | 1.55 | ≥3 | 5.31 | 149/71 | Normal | 5.45 | 127 | 4.72 | 59.46 | 14.0 4 |
| | **Mean±S D** | **18± 4** | **9.56 ±2.13** | / | **93.7 ±105.** 9 | **49±5** | **1.29 ±0.35** | **1.71 ±0.4 0** | / | **5.17 ±0.66** | **150±19/80± 17** | / | **5.78 ±0.35** | **138 ±14** | **4.81 ±0.48** | **57.44 ±11.6 7** | **9.54 ±3.2 9** |

Note: For No. 257 animal which is with thoracic deformity, cardiac ultrasound images cannot be collected.

Table 5: Baseline period-the underlying causes and characteristics of rhesus monkeys with chronic DKD renal insufficiency in each dose group (continued).

| Group | Animal No. | Age | Body weight | Kidney functiondiagnosis | UACR | eGFR | CR-S | CYSC | Years of abnormal glucose and lipid metabolism | FPG | Blood pressure (SBP/DBP) | Anemia diagnosis | RBC | HGB | $K^+$ | EF | E/Ea |
|---|---|---|---|---|---|---|---|---|---|---|---|---|---|---|---|---|---|
| | | yrs | kg | CKD-EPI rating | mg/g | (ml/ min/1.73m$^2$) | mg/dL | mg/L | | mmol/L | mmHg | | $10^{12}$/L | g/L | mmol/L | % | / |
| The group of malate of compound of Formula I 2mg/kg (n=5) | **4829** | 20 | 12.13 | G3a | 3.5 | 54 | 1.15 | 1.57 | ≥3 | 4.09 | 126/58 | Normal | 5.11 | 124 | 4.69 | 54.41 | 17.11 |
| | **6501** | 17 | 10.55 | G3a | 2.5 | 50 | 1.14 | 1.87 | ≥3 | 4.61 | 111/56 | Normal | 5.30 | 129 | 5.80 | 70.41 | 7.73 |
| | **287** | 15 | 14.53 | G3a-A2 | 15.3 | 48 | 1.36 | 1.72 | ≥3 | 6.87 | 184/78 | Normal | 5.62 | 133 | 4.77 | 57.45 | 6.69 |
| | **4721** | 18 | 10.79 | G3a-A2 | 122.1 | 46 | 1.42 | 1.69 | ≥3 | 5.53 | 143/83 | Normal | 4.98 | 119 | 4.07 | 57.90 | 17.43 |
| | **2091** | 19 | 9.88 | G3a-A2 | 29.3 | 58 | 1.06 | 1.56 | ≥3 | 7.7 | 137/74 | Normal | 6.13 | 145 | 5.40 | 62.76 | 8.89 |
| | **Mean ±SD** | **18±2** | **11.58 ± 1.84** | / | **34.5 ±50.1** | **51±5** | **1.23 ±0.16** | **1.68 ±0.13** | / | **5.76 ±1.51** | **140±27/ 70±12** | / | **5.43 ±0.46** | **130 ±10** | **4.95 ±0.67** | **60.59 ±6.25** | **11.57 ±5.26** |

(continued)

| Group | Animal No. | Age | Body weight | Kidney functiondiagnosis | UACR | eGFR | CR-S | CYSC | Years of abnormal glucose and lipid metabolism | FPG | Blood pressure (SBP/DBP) | Anemia diagnosis | RBC | HGB | $K^+$ | EF | E/Ea |
|---|---|---|---|---|---|---|---|---|---|---|---|---|---|---|---|---|---|
| | | yrs | kg | CKD-EPI rating | mg/g | (ml/min/1.73m$^2$) | mg/dL | mg/L | | mmol/L | mmHg | | $10^{12}$/L | g/L | mmol/L | % | / |
| The group of malate of compound of Formula I 1mg/kg (n=5) | **1567** | 16 | 11.47 | G3a-A2 | 32.8 | 51 | 1.25 | 1.67 | ≥3 | 5.03 | 101/54 | Normal | 5.69 | 127 | 4.50 | 65.25 | 10.50 |
| | **4861** | 18 | 9.77 | G3a-A2 | 30.0 | 54 | 1.03 | 1.80 | ≥3 | 6.09 | 105/56 | Normal | 5.69 | 127 | 4.51 | 55.89 | 9.53 |
| | **5453** | 19 | 14.93 | G3a | 2.9 | 53 | 1.09 | 1.72 | ≥3 | 5.75 | 125/60 | Normal | 5.85 | 140 | 4.40 | 54.86 | 9.22 |
| | **6653** | 20 | 10.46 | G3a | 1.6 | 50 | 1.05 | 1.84 | ≥3 | 6.29 | 121/59 | Normal | 5.49 | 124 | 4.78 | 69.87 | 8.68 |
| | **5073** | 15 | 10.34 | G3a | 4.1 | 52 | 1.21 | 1.75 | ≥3 | 4.59 | 129/70 | Normal | 5.64 | 136 | 4.79 | 47.92 | 10.36 |
| | **Mean ±SD** | **18±2** | **11.39 ±2.07** | / | **14.3 ±15.7** | **52±1** | **1.13 ±0.10** | **1.76 ±0.07** | / | **5.55 ±0.72** | **116±13 60±6** | / | **5.67 ±0.13** | **131 ±7** | **4.60 ±0.18** | **58.76 ±8.75** | **9.66 ±0.77** |

### 3. Experimental design

### 3.1 Grouping and dosage design

In the trial, animals were brought in three stages. In the first stage, there were two groups: 3 animals (No. 257, No. 4721, No. 287) in a group with 5mg/kg of the malate of compound of formula I, and 2 animals (No. 1029, No. 4713) in the placebo group. In the second stage, there were three groups: 3 animals (No. 144, No. 5073, No. 6651) in a group with 5mg/kg of the malate of compound of formula I, 3 animals (No. 2091, No. 4861, No. 6645) in the Valsartan group, and 2 animals (No. 6653, No. 231) in the placebo group. In the third stage, there were two groups: 5 animals (No. 4829, No. 6501, No. 287, No. 4721, No. 2091) in a group with 2mg/kg of the malate of compound of formula I, and 5 animals (No. 1567, No. 4861, No. 5453, No. 6653, No. 5073) in a group with 1 mg/kg of the malate of compound of formula I.

Among them, 2 animals (No. 287 and No. 4721) in the first stage were subjected to drug withdrawal and elution for about 9 months, and 4 animals (No. 5073, No. 2091, No. 4861 and No. 6653) in the second stage were subjected to drug withdrawal and elution for about 5 months, and then they entered the baseline period of the third stage (the baseline period was 1 month).

See Table 6 below for details.

Table 6: Group design information

| Group name | Number of animals | Dose (mg/kg/ time) | Dosing frequency |
|---|---|---|---|
| Placebo group | 4 | / | / |
| Valsartan group | 3 | 2.67 | once or twice a day |
| The group with 5mg/kg of the malate of compound of Formula I | 6 | 5 | Once a day |
| The group with 2mg/kg of the malate of compound of Formula I | 5 | 2 | Once a day |
| The group with 1mg/kg of the malate of compound of Formula I | 5 | 1 | Once a day |
| Remarks: In the Valsartan group, the drug was administered once a day during the administration period in the first and second weeks, and twice a day from the third week to the eighth week. | | | |

### 3.2 Dosing information

Route of administration: Oral administration.

Dosing frequency: the malate of compound of formula I was administered once a day; in the Valsartan group, the drug was administered once a day during the administration period in the first and second weeks, and twice a day from the third week to the eighth week.

Dosage calculation: the dose for the next week was calculated based on the body weight weighed each time.

Administration time: 08:00-09:00 administration.

### 3.3 Main efficacy indicators

Glomerular filtration rate $eGFR_{creat\text{-}cys}$: creatinine (Cr-P), urea nitrogen (BUN) and cystatin (CysC) were detected, and the eGFR value was obtained by calculation. It was performed once before the administration and once every two weeks after the administration.

$$eGFR_{male}=135\times\min(Cr/0.9,1)^{-0.207}\times\max(Cr/0.9,1)^{-0.601}\times\min(CysC/0.8,1)^{-0.375}\times\max(CysC/0.8,1)^{-0.711}\times0.995^{Age\times3}$$

$$eGFR_{female}=135\times\min(Cr/0.7,1)^{-0.284}\times\max(Cr/0.7,1)^{-0.601}\times\min(CysC/0.8,1)^{-0.375}\times\max(CysC/0.8,1)^{-0.711}\times0.995^{Age\times3}\times0.969$$

UACR: 4h/6h urine was collected, urine microalbumin (malb) and urine creatinine (Cr-U) were detected, and the UACR value was obtained by calculation. This was performed once before administration and once after administration. UACR=MALB / Cr-U.

Blood pressure: the blood pressure was checked after anesthesia, including systolic blood pressure (SBP), diastolic blood pressure (DBP), mean blood pressure (MBP) and heart rate (HR). This was performed once before administration and once at weeks 4 and 8 respectively after administration.

Cardiac GE Doppler ultrasound: historical cardiac ultrasonography once within 1 year (selected animals), once before administration (within 30 days (D-30) of the baseline period), and once at week 8 after administration.

### 3.4 Secondary efficacy indicators

Serum potassium, twice before administration, once every 2 weeks during the administration period;
Glucose and lipid metabolism and liver function: FPG, FRA, LDL-c, HDL-c, TG, TC, NT-proBNP, ALT, AST, TBIL, etc., twice before administration, once at weeks 4 and 8 respectively after administration;
Other biochemical indicators and hematology testing, once each before and after administration;
Body weight: once before administration, once a week during the administration period;
After administration, the changes in food intake and behavior were observed for 24 hours every day.

### 3.5 Testing methods and equipments

Testing methods: see Table 7 and Table 8 below.

Hematology testing equipment: Siemens ADVIA 2120i Hematology Systems.

Blood biochemical and urine index detection equipment: Roche cobas6000 analyzer series C501 module detection. ELISA kit was used for NT-proBNP test.

Table 7: Biochemical testing items

| Measurement item | Unit | Measurement method |
|---|---|---|
| Serum potassium ion(K+) | mmol/L | Ion selective electrode method, indirect method |
| N-terminal pro-B-type natriuretic peptide (NT-proBNP) | pg/mL | Enzyme-linked immunosorbent assay |
| Urine-Micro albumin (Malb) | g/L | Immunoturbidimetry, 2-point endpoint |
| Urine-creatinine (Cr-U) | mg/dL | Enzyme colorimetry |
| Blood urea nitrogen (BUN) | mg/dL | Colorimetric method, rate method |
| Plasma creatinine (Cr-P) | mg/dL | Enzyme colorimetry |
| Cystatin C (CysC) | mg/L | Immunoturbidimetry |
| Total cholesterol (TC) | mmol/L | Enzyme colorimetry |
| Triglycerides (TG) | mmol/L | Enzyme colorimetry |
| High density lipoprotein (HDL-c) | mmol/L | Homogeneous enzyme Colorimetry |
| Low density lipoprotein (LDL-c) | mmol/L | Homogeneous enzyme Colorimetry |
| Blood glucose (FPG) | mmol/L | Hexokinase method |
| Fructosamine (FRA) | μmol/L | Rate method |
| Alanine aminotransferase (ALT) | IU/L | IFCC rate method |
| Aspartate aminotransferase (AST) | IU/L | Colorimetry |
| Total bilirubin (TBIL) | μmol/L | Diazo method |
| Alkaline phosphatase (ALP) | IU/L | Colorimetric method, rate method |
| Total protein (TP) | g/L | Colorimetry, 2-point end point |
| Albumin (ALB) | g/L | colorimetry |
| Direct bilirubin (DBIL) | μmol/L | Diazo method, 2-point end point |
| γ -glutamyl transpeptidase (GGT) | IU/L | Enzyme colorimetry |

Table 8: Hematology test items

| Index | Unit | Measurement method |
|---|---|---|
| White blood cell count (WBC) | 10^9/L | 2D laser |
| Red blood cell count (RBC) | 10^12/L | 2D laser |
| Hemoglobin (HGB) | g/L | Cyanmethemoglobin |
| Hematocrit (HCT) | % | 2D laser |
| Mean red blood cell volume (MCV) | fL | 2D laser |
| Mean red blood cell hemoglobin (MCH) | pg | 2D laser |
| Mean red blood cell hemoglobin concentration (MCHC) | g/L | 2D laser |
| Red blood cell volume distribution width (RDW) | % | 2D laser |
| Hemoglobin distribution width (HDW) | g/L | 2D laser |
| Directly measured mean hemoglobin concentration (CHCM) | g/L | 2D laser |
| Average hemoglobin content (CH) | pg | 2D laser |
| Mean red blood cell CH distribution width (CHDW) | pg | 2D laser |
| Total platelets (PLT) | 10^9/L | 2D laser |
| Plateletcrit (PCT) | % | 2D laser |
| Platelet volume distribution width (PDW) | % | 2D laser |
| Mean platelet volume (MPV) | fL | 2D laser |

### 3.6 Cardiac Doppler Ultrasound

Anesthesia method: 15mg/kg of ketamine hydrochloride was used for intramuscular injection anesthesia. According to the animal anesthesia state, the supplemental anesthesia will be performed after judgment by veterinarian. Each supplemental anesthesia dose was 1/2 of the initial dose.

Detection method: The animal was placed in the left decubitus position, and the 6S-RS probe (frequency 2.7-8.0 MHz) was used for image acquisition.

Analysis method: After the image was collected and saved, the supporting analysis workstation EchoPAC Software was used to repeatedly detect various indicators of systolic and diastolic functions in 3 consecutive cardiac cycles.

Testing indicators and ultrasonic technology: see Table 9 below.

Testing equipment: GE Vivid S5 color Doppler ultrasound diagnostic system.

### 3.7 Blood pressure test

Test method: 15 mg/kg of ketamine hydrochloride was used for intramuscular injection to anesthetize the animal. After anesthesia, the animal was placed in a supine position. The left upper arm was shaved clean, and a suitable size cuff was tied according to the standard. The blood oxygen probe was clamped on the animal's finger or toe (except for the left hand), and the red light-sensitive surface was on the side of the finger pad. The blood pressure of the animal was continuously measured for 3 times in the automatic mode with an interval of 1 min. If the difference of DBP, SBP and MBP of blood pressure measured more than three times was not significant (the difference between the highest value and the lowest value was less than 15mmHg), the measurement was completed.

Testing indicators: testing indicators included systolic blood pressure (SBP), diastolic blood pressure (DBP), mean blood pressure (MBP) and heart rate (HR).

Testing equipment: GE B40i electrophysiological monitor.

Table 9: Cardiac ultrasound testing indicators

| Testing indicators | Unit | Testing technology |
|---|---|---|
| Left atrium anteroposterior diameter LAAD (mm) | mm | Two-dimensional ultrasound |
| Left atrium transverse diameter LATD (mm) | mm | |
| Longitudinal diameter of left atrium LAVD (mm) | mm | |
| LA Volume-Ellipsoid (ml) | ml | |

(continued)

| Testing indicators | Unit | Testing technology |
|---|---|---|
| Blood flow peak velocity of mitral valve at early diastole E | cm/s | Pulse Doppler |
| E peak deceleration time EDT | ms | |
| Blood flow peak velocity of mitral valve at late diastole A | cm/s | |
| E/A | / | |
| A duration A Dur | ms | |
| Peak Velocity of Mitral Annulus at early diastole Ea | cm/s | Tissue Doppler |
| Peak Velocity of Mitral Annulus at late diastole Aa | cm/s | |
| Ea/Aa | / | |
| E/Ea | / | |
| Isovolumic diastolic time IVRT | ms | |
| Isovolumic contraction time IVCT | ms | |
| Pulmonary vein S wave peak velocity PV S | cm/s | Pulse Doppler |
| Pulmonary vein D wave peak velocity PV D | cm/s | |
| Pulmonary venous reflux Ar wave velocity PV Ar | cm/s | |
| Pulmonary venous reflux Ar wave duration Ar Dur | ms | |
| Ar-A Dur | ms | |
| Blood flow propagation velocity of left ventricle at early diastole Vp | cm/s | Color M-mode ultrasound |
| Four-chamber heart end-diastolic volume LVEDV MOD A4C | ml | Two-dimensional biplane Simpson method |
| Four-chamber heart end-diastolic left ventricular long diameter LVLd A4C | mm | |
| Four-chamber heart end-systolic volume LVESV MOD A4C | ml | |
| Four-chamber heart end-systolic left ventricular long diameter LVLs A4C | mm | |
| Four-chamber heart stroke volume SV MOD A4C | ml | |
| Four-chamber cardiac ejection fraction LVEF MOD A4C | % | |
| Two-chamber heart end-diastolic volume LVEDV MOD A2C | ml | |
| Two-chamber heart end-diastolic left ventricular long diameter LVLd A2C | mm | |
| Two-chamber heart end-systolic volume LVESV MOD A2C | ml | |
| Two-chamber heart end-systolic left ventricular long diameter LVLs A2C | mm | |
| Two-chamber heart stroke volume SV MOD A2C | ml | |
| Two-chamber cardiac ejection fraction LVEF MOD A2C | % | |
| Biplane end-systolic volume LVESV MOD BP | ml | |
| Biplane end-diastolic volume LVEDV MOD BP | ml | |
| Biplane ejection fraction EF Biplane | % | |
| Biplane output per minute CO Biplane | L/min | |
| Heart rate HR | BPM | |
| RR interval R-R | ms | |

### 3.8 Clinical observation

Number of observations: Observation was performed once a day.

Observation method: observation in cage.

Observation content: injection site, skin, hair, eyes, ears, nose, oral cavity, chest, abdomen, urogenital, limbs and other parts, as well as breathing, exercise, urinary, defecation and behavior changes.

### 3.9 Determination of food intake

Feeding method: Food was supplied once at 8:00-9:00 in the morning, 10:00 in the morning, 2:00 in the afternoon, and 4:00 in the afternoon, respectively, to ensure that there was food in the food box and animals ate freely. The remaining feed was removed at 07:40-8:00 in the next day.

Feeding amount: about 250-500 g/animal/day. In the experiment, it was ensured that there was feed in the food box for 24 hours, and the animals can eat freely.

Method for measuring food intake: The feed was estimated by a semi-quantitative method, and the amount of food given, the amount discarded and the remaining amount in food boxes were recorded every day. Food intake = food given - amount discarded - remaining amount in food box.

### 3.10 Weight determination

Weighing time: before feeding on the day.

Determination method: The animal was fasted for 14-16 h before weighing, and the animal was awake, after transferred to the transfer cage, it was weighed with a large animal scale.

Measuring instrument: METTLER TOLEDO electronic platform scale.

### 4.0 Criteria for termination of the test

a. Serious adverse events occurred.
b. A serious infection occurred.

## 5. Results and analysis

### 5.1 Effects on UACR

The effects of administration of each group on proteinuria (UACR) were shown in Table 10 below. In each group, the CKD rhesus monkeys who had moderate to severe increase in proteinuria, manifested by baseline urine albumin/creatinine ratio (UACR) ≥ 15mg/g to 350mg/g (4-6 hours urine collection), were chosen, and the change of albumin/creatinine ratio from baseline to 8-week administration (W8) was analyzed.

**Placebo group** (n=4): Compared with the baseline, the UACR of W8 measured at the end of the trial period was increased by 30.64±34.50% on average. The UACR of the proteinuria from 4 cases of animals was fluctuated steadily within a certain range, which did not show rapid progress.

**Valsartan group** (n=3, moderate to severe proteinuria increase was included in the statistics): Compared with the baseline, the UACR from 2 animals (No. 2091, No. 4861) of W8 was decreased by 40%-70%, and both showed benefit. For one animal, the baseline UACR was 15 mg/g, and the W8 UACR after administration was 20.4%, which showed the benefit was not obvious. Due to the small number of animals in the group, there was no statistical difference compared with the placebo group.

**The group of the malate of compound of Formula I (5 mg/kg once a day (qd))** (n=6, 4/6 moderate-to-severe proteinuria increase was included in statistics): Compared with the baseline, the W8 UACR after administration was decreased by 46.87±36.93% on average, which was significantly lower than that in the placebo group ($p<0.05$). Among them, the UACR from 3 animals of W8 on was decreased by 60%-70%, as compared with the baseline, and all showed benefits. One animal showed no benefit.

**The group of the malate of compound of formula I (2 mg/kg qd)** (n=5, 3/5 moderate to severe proteinuria increase was included in the statistics): Compared with the baseline, the W8 UACR after administration was decreased by 66.00±17.42% on average, which was significantly lower than that in the placebo group ($p<0.01$). Among them, the UACR from three animals of W8 was decreased by about 50%-80% as compared with the baseline, and they all showed benefits.

**The group of the malate of compound of formula I (1 mg/kg qd)** (n=5, 2/5 moderate-to-severe proteinuria increase was included in statistics): Compared with the baseline, the UACR from one animal of W8 was decreased by 39%. The benefit was not obvious in one case.

**In short, the proteinuria (UACR) can be significantly improved by administering 2-5 mg/kg qd** of the malate of compound of formula I for 8 weeks.

Table 10: Effect of the malate of compound of formula I administered for 8 weeks on the UACR of rhesus monkey with spontaneous chronic kidney disease (CKD)

| Group | Animal No. | MALB (mg/L) | | Cr-U (mg/dL) | | Urine-Vol (ml) | | UACR (mg/g) | | UACR change rate (%) | UACR Benefit assessm ent |
|---|---|---|---|---|---|---|---|---|---|---|---|
| | | Baseline | 8 weeks | Baseline | 8 weeks | Baseline | 8 weeks | Baseline | 8 weeks | | |
| Placebo group (n=4) | 6653 | 38.3 | 4.7 | 137.74 | 15.66 | 60 | 35 | **27.8** | **30.0** | 7.94 | No benefit |
| | 231 | 10.6 | 16.6 | 73.27 | 68.31 | 115 | 210 | **14.5** | **24.3** | 67.97 | No benefit |
| | 1029 | 4.4 | 2.2 | 42.00 | 81.64 | 265 | 55 | **10.5** | **10.0** | -4.55 | No benefit |
| | 4713 | 4.2 | 13.6 | 49.45 | 105.90 | 315 | 50 | **8.5** | **12.8** | 51.20 | No benefit |
| | Mean±S D | 14.4±16.2 | 9.3±6.9 | 75.62±43.51 | 67.88±38.13 | 189±121 | 88±82 | **15.3±8.7** | **19.3±9.5** | **30.64±34.50** | / |
| Valsartan group (n=3) | 2091 | 7.1 | 6.2 | 19.02 | 28.20 | 185 | 240 | **37.3** | **22.0** | -41.10 | Benefit |
| | 4861 | 60.0 | 39.9 | 94.34 | 206.27 | 20 | 80 | **63.6** | **19.3** | -69.59 | Benefit |
| | 6645 | 25.8 | 19.5 | 171.64 | 95.47 | 20 | 37 | **15.0** | **20.4** | 35.88 | / |
| | Mean±S D | 31.0±26.8 | 21.9±17.0 | 95.00±76.31 | 109.98±89.9 2 | 75±95 | 119±107 | **38.7±24.3** | **20.6±1.3** | -24.93±54.5 6 | / |
| The group with 5mg/kg of the malate of compound of Formula I (n=6) | 144 | 93.2 | 72.4 | 78.63 | 52.32 | 30 | 60 | **118.5** | **138.4** | 16.75 | No benefit |
| | 5073 | 4.3 | 1.2 | 40.84 | 21.19 | 235 | 400 | **10.5** | **5.7** | NA | / |
| | 6651 | 46.5 | 3.4 | 182.87 | 37.25 | 30 | 90 | **25.4** | **9.1** | -64.10 | Benefit |
| | 257 | 59.3 | 112.3 | 21.23 | 128.11 | 208 | 25 | **279.3** | **87.7** | -68.62 | Benefit |
| | 287 | 2.1 | 0.3 | 89.17 | 16.98 | 165 | 380 | **2.4** | **1.8** | NA | / |
| | 4721 | 26.0 | 21.6 | 20.66 | 61.60 | 405 | 115 | **125.8** | **35.1** | -72.14 | Benefit |
| | Mean±S D | 38.6±35.0 | 35.2±46.7 | 72.23±61.34 | 52.91±40.68 | 179±141 | 178±167 | **93.7±105.9** | **46.3±55.4** | -46.87±36.9 3# | / |

Note: 1. UACR=MALB / Cr-U
2. Change rate = (time point after administration - baseline value)/baseline value * 100%, negative value represented decrease; "#" compared with placebo group $p<0.05$.
3. NA, UACR normal animals were not included in the statistics.

Table 10: Effect of the malate of compound of formula I administered for 8 weeks on the UACR of rhesus monkey with spontaneous chronic kidney disease (CKD) (continued)

| Group | Anim al No. | MALB (mg/L) | | | Cr-U (mg/dL) | | | Urine-Vol (ml) | | | UACR (mg/g) | | | UACR CHANGE RATE (%) | UACR Benefi t assess ment |
|---|---|---|---|---|---|---|---|---|---|---|---|---|---|---|---|
| | | Baselin e | 4 weeks | 8 weeks | Baseline | 4 weeks | 8 weeks | Baselin e | 4 weeks | 8 weeks | Baselin e | 4 weeks | 8 weeks | | |
| The group with 2mg/kg of the malate of compoun d of Formula I (n=5) | 4829 | 1.2 | 1.0 | 3.0 | 33.98 | 32.18 | 53.10 | 290 | 330 | 77 | 3.5 | 3.1 | 5.6 | NA | / |
| | 6501 | 0.6 | 12.5 | 0.4 | 24.39 | 157.85 | 33.60 | 315 | 20 | 260 | 2.5 | 7.9 | 1.2 | NA | / |
| | 287 | 5.3 | 5.3 | 0.6 | 34.58 | 41.80 | 26.33 | 53 | 320 | 420 | 15.3 | 12.7 | 2.3 | -85.13 | Benefi t |
| | 4721 | 145.4 | 103.7 | 69.5 | 119.07 | 111.58 | 116.29 | 55 | 60 | 60 | 122.1 | 92.9 | 59.8 | -51.06 | Benefi t |
| | 2091 | 6.8 | 2.4 | 3.6 | 23.24 | 66.89 | 32.22 | 370 | 85 | 265 | 29.3 | 3.6 | 11.2 | -61.81 | Benefi t |
| | Mean ±SD | 31.9 ±63.5 | 25.0 ±44.2 | 15.4 ±30.3 | 47.05 ±40.60 | 82.06 ±52.33 | 52.31 ±37.16 | 217 ±151 | 163 ±150 | 216 ±150 | 34.5 ±50.1 | 24.0 ±38.7 | 16.0 ±24.8 | -66.00 ±17.42## | / |
| The group with 1mg/kg of the malate of compoun d of Formula I Cn=5) | 1567 | 9.5 | 4.6 | 5.2 | 28.93 | 30.74 | 26.02 | 393 | 170 | 375 | 32.8 | 15.0 | 20.0 | -39.14 | **Benefi** t |
| | 4861 | 26.8 | 34.1 | 38.4 | 89.48 | 121.39 | 118.23 | 55 | 35 | 25 | 30.0 | 28.1 | 32.5 | 8.44 | No benefit |
| | 5453 | 0.6 | 1.4 | 0.8 | 20.69 | 19.36 | 25.52 | 440 | 380 | 470 | 2.9 | 7.2 | 3.1 | NA | / |
| | 6653 | 0.9 | 5.1 | 7.6 | 56.97 | 108.29 | 290.17 | 120 | 310 | 25 | 1.6 | 4.7 | 2.6 | NA | / |
| | 5073 | 0.9 | 1.3 | 1.1 | 21.90 | 16.09 | 17.93 | 443 | 470 | 520 | 4.1 | 8.1 | 6.1 | NA | / |
| | Mean ±SD | 7.7 ±11.3 | 9.3 ±14.0 | 10.6 ±15.8 | 43.59 ±29.56 | 59.17 ±51.32 | 95.57 ±116.3 6 | 290 ±187 | 273 ±172 | 283 ±241 | 14.3 ±15.7 | 12.6 ±9.4 | 12.9 ±13.0 | -15.35 ±33.65 | / |

Note: 1. UACR=MALB / Cr-U

2. Change rate = (time point after administration - baseline value)/baseline value*100%, negative value represented decrease; "#" compared with placebo group $p<0.05$, "##" compared with placebo group $p<0.01$.

3. NA, UACR normal animals were not included in the statistics.

### 5.2 Effect on glomerular filtration rate $eGFR_{creat-cys}$

The effects on eGFR in each group were shown in Table 11 and Table 12 below. Selected **$eGFR_{creat-cys}$** in each group: 30-59 ml/min/1.73m$^2$. The changes in the glomerular filtration rate of W8 and baseline were analyzed.

**Placebo group** (n=4): Compared with the baseline, the glomerular filtration rate **$eGFR_{creat-cys}$** from 4 cases of animal was fluctuated stably within a certain range and no rapid progress was shown.

**Valsartan group** (n=3): Compared with the baseline, the glomerular filtration rate **$eGFR_{creat-cys}$** of W8 in the Valsartan group was increased by 6±2 ml/min/1.73m$^2$ on average, which was a very significant increase compared with the change value of the placebo group ( $p<0.01$). The glomerular deterioration was significantly improved, and the curative effect was time-dependent.

**The group of the malate of compound of Formula** I (5 **mg/kg qd)** (n=6): Compared with the baseline, the glomerular filtration rate **$eGFR_{creat-cys}$** of W8 was increased by 6±6 ml/min/1.73m$^2$ on average, which was a significant increase compared with the change value of the placebo group ($p<0.05$). The curative effect was time-dependent.

**The group of the malate of compound of Formula I (2 mg/kg qd)** (n=5): Compared with the baseline, the glomerular filtration rate **$eGFR_{creat-cys}$** of W8 was increased by 3±3 ml/min/1.73m$^2$ on average, which was a significant increase compared with the change value of the placebo group ($p<0.05$).

**The group of the malate of compound of Formula I (1 mg/kg qd)** (n=5): Compared with the baseline, the glomerular filtration rate **$eGFR_{creat-cys}$** of W8 was increased by 3±4 ml/min/1.73m$^2$ on average. Compared with the change value of the placebo group, no statistical change was shown.

**In short, the glomerular filtration rate $eGFR_{creat-cys}$ can be significantly improved by administering 2-5mg/kg qd** of the malate of compound of formula I for 8 weeks.

Table 11: Effect of the malate of compound of formula I on **$eGFR_{creat-cys}$** for 8-week administration in rhesus monkeys with spontaneous chronic kidney disease

| Group | Animal No. | $eGFR_{creat-cys}$ (ml/min/1.73m$^2$) | | | | | $eGFR_{creat-cys}$ Change value (8 weeks) |
|---|---|---|---|---|---|---|---|
| | | Baseline | 2 weeks | 4 weeks | 6 weeks | 8 weeks | |
| Placebo group (n=4) | 6653 | 47 | 49 | 43 | 47 | 44 | -3 |
| | 231 | 58 | 62 | 57 | 56 | 58 | 1 |
| | 1029 | 53 | 57 | 55 | 50 | 56 | 2 |
| | 4713 | 47 | 49 | 43 | 46 | 45 | -2 |
| | Mean±SD | 51±5 | 54±6 | 49±8 | 50±4 | 51±8 | -1±3 |
| Valsartan group (n=3 ) | 2091 | 55 | 58 | 55 | 55 | 60 | 5 |
| | 4861 | 50 | 54 | 57 | 55 | 59 | 8 |
| | 6645 | 40 | 46 | 46 | 47 | 46 | 5 |
| | Mean±SD | 48±7 | 53±6 | 53±6 | 53±5 | 55±8 | 6±2## |
| The group with 5mg/kg of the malate of compound of Formula I (n=6) | 144 | 55 | 51 | 52 | 53 | 57 | 3 |
| | 5073 | 48 | 57 | 51 | 54 | 53 | 6 |
| | 6651 | 51 | 62 | 57 | 49 | 55 | 4 |
| | 257 | 41 | 37 | 39 | 40 | 43 | 2 |
| | 287 | 53 | 54 | 65 | 64 | 61 | 8 |
| | 4721 | 43 | 61 | 55 | 54 | 60 | 17 |
| | Mean±SD | 49±5 | 54±9 | 53±8 | 53±8 | 55±7 | 6±6# |
| The group with 2mg/kg of the malate of | 4829 | 54 | 58 | 61 | 58 | 59 | 5 |
| | 6501 | 50 | 47 | 45 | 48 | 50 | 0 |
| | 287 | 48 | 46 | 50 | 48 | 51 | 2 |
| | 4721 | 46 | 43 | 56 | 51 | 52 | 6 |
| | 2091 | 58 | 57 | 59 | 62 | 59 | 1 |

(continued)

| Group | Animal No. | eGFR$_{creat-cys}$ (ml/min/1.73m$^2$) | | | | | eGFR$_{creat-cys}$ Change value (8 weeks) |
|---|---|---|---|---|---|---|---|
| | | Baseline | 2 weeks | 4 weeks | 6 weeks | 8 weeks | |
| compound of Formula I (n=5 ) | Mean±SD | 51±5 | 50±7 | 54±7 | 54±6 | 54±4 | 3±3# |
| The group with 1mg/kg of the malate of compound of Formula I (n=5 ) | 1567 | 51 | 53 | 60 | 58 | 58 | 7 |
| | 4861 | 54 | 56 | 64 | 57 | 59 | 6 |
| | 5453 | 53 | 50 | 55 | 56 | 53 | 0 |
| | 6653 | 50 | 48 | 52 | 53 | 48 | -2 |
| | 5073 | 52 | 56 | 56 | 57 | 57 | 5 |
| | Mean±SD | 52±1 | 52±3 | 57±5 | 56±2 | 55±5 | 3±4 |
| Note: Change value = 8 weeks - Baseline. Negative value represented decrease. "#" change value compared with placebo group p<0.05, "##" change value compared with placebo group p<0.01. | | | | | | | |

Table 12: Changes of **eGFR$_{creat-cys}$** in rhesus monkeys with spontaneous chronic kidney disease after 8-week administration of the malate of compound of formula I

| Group | Animal No. | eGFR$_{creat-cys}$ Change value (ml/min/1.73m$^2$) | | | | |
|---|---|---|---|---|---|---|
| | | Baseline | 2 weeks | 4 weeks | 6 weeks | 8 weeks |
| Placebo group (n=4) | 6653 | 0 | 2 | -4 | 0 | -3 |
| | 231 | 0 | 4 | -1 | -2 | 1 |
| | 1029 | 0 | 4 | 2 | -4 | 2 |
| | 4713 | 0 | 2 | -4 | -1 | -2 |
| | Mean±SD | 0±0 | 3±1 | -2±3 | -2±2 | -1±3 |
| Valsartan group (n=3) | 2091 | 0 | 4 | 1 | 1 | 5 |
| | 4861 | 0 | 4 | 7 | 5 | 8 |
| | 6645 | 0 | 6 | 6 | 7 | 5 |
| | Mean±SD | 0±0 | 5±1 | 5±3 | 4±3 | 6±2 |
| The group with 5mg/kg of the malate of compound of Formula I (n=6) | 144 | 0 | -4 | -3 | -1 | 3 |
| | 5073 | 0 | 9 | 4 | 6 | 6 |
| | 6651 | 0 | 11 | 6 | -2 | 4 |
| | 257 | 0 | -4 | -2 | -1 | 2 |
| | 287 | 0 | 1 | 12 | 11 | 8 |
| | 4721 | 0 | 17 | 11 | 11 | 17 |
| | Mean±S | 0±0 | 5±9 | 5±6 | 4±6 | 6±6 |
| The group with 2mg/kg of the malate of compound of Formula I (n=5 ) | 4829 | 0 | 5 | 7 | 4 | 5 |
| | 6501 | 0 | -3 | -5 | -1 | 0 |
| | 287 | 0 | -3 | 1 | 0 | 2 |
| | 4721 | 0 | -3 | 10 | 5 | 6 |
| | 2091 | 0 | 0 | 1 | 5 | 1 |
| | Mean±SD | 0±0 | -1±3 | 3±6 | 3±3 | 3±3 |

(continued)

| Group | Animal No. | eGFR$_{creat-cys}$ Change value (ml/min/1.73m$^2$) | | | | |
|---|---|---|---|---|---|---|
| | | Baseline | 2 weeks | 4 weeks | 6 weeks | 8 weeks |
| The group with 1mg/kg of the malate of compound of Formula I (n=5 ) | 1567 | 0 | 1 | 8 | 7 | 7 |
| | 4861 | 0 | 2 | 10 | 3 | 6 |
| | 5453 | 0 | -3 | 3 | 3 | 0 |
| | 6653 | 0 | -2 | 1 | 3 | -2 |
| | 5073 | 0 | 4 | 5 | 6 | 5 |
| | Mean±SD | 0±0 | 0±3 | 5±4 | 4±2 | 3±4 |
| Note: Change value = current value - Baseline, negative value represented decrease. | | | | | | |

### 5.3 Effects on CysC, Cr-P and BUN

The effects on CysC, Cr-P and BUN after administration in each group were shown in Tables 13 to 15.

Table 13: Effect of the malate of compound of formula I on Cr-P in rhesus monkeys with spontaneous chronic kidney disease for 8-week administration

| Group | Animal No. | Cr-P (mg/dL) | | | | |
|---|---|---|---|---|---|---|
| | | Baseline | 2 weeks | 4 weeks | 6 weeks | 8 weeks |
| Placebo group (n=4) | 6653 | 1.33 | 1.23 | 1.19 | 1.28 | 1.16 |
| | 231 | 1.20 | 1.19 | 1.11 | 1.18 | 1.20 |
| | 1029 | 0.98 | 1.00 | 0.92 | 1.00 | 1.04 |
| | 4713 | 1.33 | 1.42 | 1.25 | 1.47 | 1.33 |
| | Mean±SD | 1.21±0.17 | 1.21±0.17 | 1.12±0.14 | 1.23±0.20 | 1.18±0.12 |
| Valsartan group (n=3 ) | 2091 | 1.28 | 1.12 | 1.12 | 1.11 | 1.09 |
| | 4861 | 1.31 | 1.12 | 1.08 | 1.11 | 1.03 |
| | 6645 | 1.50 | 1.22 | 1.26 | 1.26 | 1.26 |
| | Mean±SD | 1.36±0.12 | 1.15±0.06* | 1.15±0.09** | 1.16±0.09** | 1.13±0.12** |
| The group with 5mg/kg of the malate of compound of Formula I (n=6) | 144 | 0.90 | 0.92 | 0.85 | 0.87 | 0.85 |
| | 5073 | 1.53 | 1.14 | 1.26 | 1.42 | 1.22 |
| | 6651 | 1.10 | 0.88 | 0.89 | 1.02 | 0.95 |
| | 257 | 0.99 | 1.12 | 1.15 | 1.06 | 1.04 |
| | 287 | 1.43 | 1.40 | 1.13 | 1.12 | 1.11 |
| | 4721 | 1.79 | 0.96 | 1.33 | 1.32 | 1.18 |
| | Mean±SD | 1.29±0.35 | 1.07±0.19 | 1.10±0.19* | 1.14±0.20 | 1.06±0.14* |
| The group with 2mg/kg of the malate of compound of Formula I (n=5 ) | 4829 | 1.15 | 1.1 | 1.15 | 1.17 | 1.17 |
| | 6501 | 1.14 | 1.19 | 1.44 | 1.27 | 1.29 |
| | 287 | 1.36 | 1.52 | 1.47 | 1.43 | 1.4 |
| | 4721 | 1.42 | 1.54 | 1.26 | 1.34 | 1.3 |
| | 2091 | 1.06 | 1.11 | 1.09 | 1.04 | 1.11 |
| | Mean±SD | 1.23±0.16 | 1.29±0.22 | 1.28±0.17 | 1.25±0.15 | 1.25±0.11 |

(continued)

| Group | Animal No. | Cr-P (mg/dL) | | | | |
|---|---|---|---|---|---|---|
| | | Baseline | 2 weeks | 4 weeks | 6 weeks | 8 weeks |
| The group with 1mg/kg of the malate of compound of Formula I (n=5 ) | 1567 | 1.25 | 1.21 | 1.11 | 1.16 | 1.14 |
| | 4861 | 1.03 | 1.07 | 1.01 | 1.07 | 1.01 |
| | 5453 | 1.09 | 1.12 | 1.12 | 1.07 | 1.17 |
| | 6653 | 1.05 | 1.13 | 1.06 | 1.07 | 1.18 |
| | 5073 | 1.21 | 1.24 | 1.26 | 1.22 | 1.22 |
| | Mean±SD | 1.13±0.10 | 1.15±0.07 | 1.11±0.09 | 1.12±0.07 | 1.14±0.08 |
| Note: "*" p<0.05 compared with the baseline value, "**" p<0.01 compared with the baseline value. | | | | | | |

Table 14: Effect of the malate of compound of formula I on CysC in rhesus monkeys with spontaneous chronic kidney disease for 8-week administration

| Group | Animal No. | CysC (mg/L) | | | | |
|---|---|---|---|---|---|---|
| | | Baseline | 2 weeks | 4 weeks | 6 weeks | 8 weeks |
| Placebo group (n=4) | 6653 | 2.03 | 1.80 | 1.74 | 1.99 | 1.88 |
| | 231 | 1.42 | 1.42 | 1.37 | 1.47 | 1.49 |
| | 1029 | 1.59 | 1.71 | 1.66 | 1.63 | 1.83 |
| | 4713 | 1.44 | 1.55 | 1.61 | 1.69 | 1.67 |
| | Mean±SD | 1.62±0.28 | 1.62±0.17 | 1.60±0.16 | 1.70±0.22 | 1.72±0.18 |
| Valsartan group (n=3) | 2091 | 1.66 | 1.44 | 1.47 | 1.58 | 1.59 |
| | 4861 | 1.68 | 1.62 | 1.65 | 1.58 | 1.64 |
| | 6645 | 1.73 | 1.76 | 1.74 | 1.69 | 1.63 |
| | Mean±SD | 1.69±0.04 | 1.61±0.16 | 1.62±0.14 | 1.62±0.06 | 1.62±0.03 |
| The group with 5mg/kg of the malate of compound of | 144 | 1.49 | 1.63 | 1.68 | 1.59 | 1.46 |
| | 5073 | 1.63 | 1.63 | 1.73 | 1.47 | 1.69 |
| | 6651 | 1.61 | 1.45 | 1.64 | 1.80 | 1.66 |
| | 257 | 2.52 | 2.63 | 2.41 | 2.45 | 2.29 |
| Formula I (n=6) | 287 | 1.47 | 1.46 | 1.36 | 1.40 | 1.50 |
| | 4721 | 1.55 | 1.64 | 1.44 | 1.46 | 1.39 |
| | Mean±SD | 1.71±0.40 | 1.74±0.44 | 1.71±0.37 | 1.70±0.40 | 1.67±0.33 |
| The group with 2mg/kg of the malate of compound of Formula I (n=5 ) | 4829 | 1.57 | 1.44 | 1.31 | 1.39 | 1.36 |
| | 6501 | 1.87 | 1.97 | 1.77 | 1.76 | 1.68 |
| | 287 | 1.72 | 1.71 | 1.56 | 1.65 | 1.58 |
| | 4721 | 1.69 | 1.73 | 1.43 | 1.54 | 1.55 |
| | 2091 | 1.56 | 1.51 | 1.47 | 1.42 | 1.47 |
| | Mean±SD | 1.68±0.13 | 1.67±0.21 | 1.51±0.17** | 1.55±0.16** | 1.53±0.12** |

(continued)

| Group | Animal No. | CysC (mg/L) | | | | |
|---|---|---|---|---|---|---|
| | | Baseline | 2 weeks | 4 weeks | 6 weeks | 8 weeks |
| The group with 1mg/kg of the malate of compound of Formula I (n=5 ) | 1567 | 1.67 | 1.66 | 1.49 | 1.48 | 1.51 |
| | 4861 | 1.80 | 1.64 | 1.43 | 1.61 | 1.59 |
| | 5453 | 1.72 | 1.83 | 1.57 | 1.62 | 1.62 |
| | 6653 | 1.84 | 1.83 | 1.76 | 1.68 | 1.77 |
| | 5073 | 1.75 | 1.55 | 1.50 | 1.50 | 1.51 |
| | Mean±SD | 1.76±0.07 | 1.70±0.12 | 1.55±0.13** | 1.58±0.08** | 1.60±0.11** |
| Note: "**" p<0.01 compared with the baseline value. | | | | | | |

Table 15: Effect of the malate of compound of formula I on BUN of rhesus monkeys with spontaneous chronic kidney disease for 8-week administration

| Group | Animal No. | BUN (mg/dL) | | |
|---|---|---|---|---|
| | | Baseline2 | 4 weeks | 8 weeks |
| Placebo group (n=4) | 6653 | 34.8 | 30.0 | 26.6 |
| | 231 | 34.0 | 29.3 | 34.5 |
| | 1029 | 27.5 | 33.3 | 27.9 |
| | 4713 | 36.7 | 37.4 | 38.6 |
| | Mean±SD | 33.3±4.0 | 32.5±3.7 | 31.9±5.6 |
| Valsartan group (n=3) | 2091 | 19.0 | 22.2 | 19.5 |
| | 4861 | 25.4 | 29.1 | 33.4 |
| | 6645 | 31.2 | 33.4 | 26.0 |
| | Mean±SD | 25.2±6.1 | 28.2±5.7 | 26.3±7.0 |
| The group with 5mg/kg of the malate of compound of Formula I (n=6) | 144 | 18.6 | 23.6 | 18.6 |
| | 5073 | 37.3 | 32.5 | 30.4 |
| | 6651 | 41.3 | 32.6 | 30.1 |
| | 257 | 77.3 | 62.5 | 62.5 |
| | 287 | 18.1 | 27.2 | 27.9 |
| | 4721 | 81.2 | 53.8 | 41.9 |
| | Mean±SD | 45.6±27.7 | 38.7±15.7 | 35.2±15.3 |
| The group with 2mg/kg of the malate of compound of Formula I (n=5) | 4829 | 37.5 | 42.7 | 36.4 |
| | 6501 | 24.2 | 37.1 | 29.4 |
| | 287 | 18.7 | 22.9 | 23.6 |
| | 4721 | 42.1 | 41.6 | 49.5 |
| | 2091 | 25.4 | 30.4 | 22.6 |
| | Mean±SD | 29.6±9.8 | 34.9±8.3 | 32.3±11.1 |
| The group with 1mg/kg of the malate of compound of Formula I (n=5 ) | 1567 | 35.1 | 38.0 | 29.5 |
| | 4861 | 32.4 | 24.9 | 30.5 |
| | 5453 | 19.3 | 15.4 | 16.2 |
| | 6653 | 39.8 | 36.3 | 38.5 |
| | 5073 | 40.8 | 32.7 | 28.1 |
| | Mean±SD | 33.5±8.6 | 29.5±9.3 | 28.6±8.0 |

### 5.4 Effect on blood pressure

See Table 16 for the effects of administration of each administration group on blood pressure. The blood pressure test of primates required anesthesia, so it was necessary to control the variability of heart rate at various time points to make statistics.

**Placebo group** (n=4, 2/4 hypertension): Compared with the baseline, the blood pressure of 4 cases of animals was fluctuated steadily within a certain range.

**Valsartan group** (n=3, 1/3 hypertension): Compared with the baseline, SBP and DBP were decreased by 14±4 mmHg and 7±0 mmHg on average respectively after W8 administration, which was extremely significantly lower than the change value of the placebo group ($p<0.01$).

**The group of the malate of compound of Formula I** (5 mg/kg qd) (n=6, 4/6 cases of hypertension): Compared with the baseline, SBP and DBP were reduced by 21±8 mmHg and 10±7 mmHg on average respectively after W8 administration, which was extremely significantly lower than the change value of the placebo group ($p<0.01$). The curative effect was time-dependent.

**The group of the malate of compound of Formula I (2 mg/kg qd)** (n=5, 2/5 hypertension): Compared with the baseline, SBP and DBP were reduced by 19±9 mmHg and 9±8 mmHg on average respectively after W8 administration, which was extremely significantly lower than the change value of the placebo group ($P<0.01$). The curative effect was time-dependent.

**The group of the malate of compound of Formula I (1 mg/kg qd)** (n=5): Compared with the baseline, SBP and DBP were reduced by 11±4 mmHg and 5±4 mmHg on average respectively after W8 administration, which was significantly lower than the change value of the placebo group ($p<0.05$).

**In a word,** the malate of compound of formula I showed **the activity of lowering the blood pressure when administering 1-5 mg/kg qd** for 8 weeks.

Table 16: Effect of the malate of compound of formula I on blood pressure in rhesus monkeys with spontaneous chronic kidney disease for 8-week administration

| Group | Animal No. | SBP (mmHg) | | | SBP Change value (mmHg) | | DBP (mmHg) | | | DBP Change value (mmHg) | |
|---|---|---|---|---|---|---|---|---|---|---|---|
| | | Baseline | 4 weeks | 8 weeks | 4 weeks | 8 weeks | Baseline | 4 weeks | 8 weeks | 4 weeks | 8 weeks |
| Placebo group (n=4) | 6653 | 103 | 110 | 121 | 6 | 18 | 53 | 58 | 65 | 5 | 13 |
| | 231 | 134 | 122 | 139 | -11 | 6 | 63 | 61 | 66 | -1 | 3 |
| | 1029 | 162 | 169 | 156 | 7 | -6 | 72 | 73 | 87 | 4 | 13 |
| | 4713 | 143 | 164 | 154 | 21 | 11 | 65 | 72 | 68 | 7 | 2 |
| | Mean ±SD | 135± 24 | 141± 30 | 143± 16 | 6± 13 | 7± 10 | 63± 8 | 67± 9 | 72± 11 | 4± 4 | 8± 7 |
| Valsartan group (n=3) | 2091 | 131 | 130 | 121 | -1 | -10 | 67 | 62 | 61 | -5 | -6 |
| | 4861 | 125 | 106 | 107 | -19 | -18 | 60 | 53 | 53 | -7 | -7 |
| | 6645 | 148 | 138 | 133 | -10 | -15 | 82 | 62 | 75 | -20 | -7 |
| | Mean ±SD | 135± 12 | 125± 17 | 120± 13* | -10± 9 | -14± 4## | 70± 11 | 59± 5 | 63± 11** | -10± 8# | -7± 0## |
| The group with **5mg/kg** of the malate of compound of Formula I (n=6) | 144 | 132 | 136 | NA | 3 | NA | 63 | 61 | NA | -2 | NA |
| | 5073 | 159 | 124 | NA | -35 | NA | 105 | 70 | NA | -35 | NA |
| | 6651 | 127 | NA | 114 | NA | -12 | 74 | NA | 57 | NA | -18 |
| | 257 | 180 | 159 | 161 | -21 | -19 | 97 | 81 | 97 | -16 | 0 |
| | 287 | 154 | 144 | 121 | -9 | -32 | 70 | 68 | 60 | -3 | -11 |
| | 4721 | 149 | 139 | 128 | -10 | -21 | 71 | 68 | 59 | -3 | -13 |
| | Mean ±SD | 150± 19 | 138± 12 | 129± 16** | -14± 14# | -21± 8## | 80± 17 | 71± 7 | 66± 15* | -12± 14# | -10± 7## |

(continued)

| Group | Animal No. | SBP (mmHg) | | | SBP Change value (mmHg) | | DBP (mmHg) | | | DBP Change value (mmHg) | |
|---|---|---|---|---|---|---|---|---|---|---|---|
| | | Baseline | 4 weeks | 8 weeks | 4 weeks | 8 weeks | Baseline | 4 weeks | 8 weeks | 4 weeks | 8 weeks |
| The group with **2mg/kg** of the malate of compound of Formula I (**n=5**) | 4829 | 126 | 110 | 102 | -16 | -24 | 58 | 55 | 52 | -2 | -6 |
| | 6501 | 111 | NA | NA | NA | NA | 56 | NA | NA | NA | NA |
| | 287 | 184 | 163 | 160 | -21 | -24 | 78 | 83 | 70 | -5 | -8 |
| | 4721 | 143 | NA | 121 | NA | -22 | 83 | NA | 62 | NA | -21 |
| | 2091 | 137 | 138 | 133 | 1 | -5 | 74 | 66 | 73 | -8 | -1 |
| | Mean ±SD | 140± 27 | 140± 20 | 128± 21 | -12± 11 | -19± 9## | 70± 12 | 71± 11 | 64± 8 | -2± 7 | -9± 8## |
| The group with **1mg/kg** of the malate of compound of Formula I (**n=5**) | 1567 | 101 | 104 | 94 | 4 | -7 | 54 | 51 | 53 | -3 | 0 |
| | 4861 | 105 | 109 | 89 | 4 | -15 | 56 | 54 | 49 | -2 | -8 |
| | 5453 | 125 | 124 | 114 | -1 | -12 | 60 | 65 | 53 | 5 | -7 |
| | 6653 | 121 | NA | NA | NA | NA | 59 | NA | NA | NA | NA |
| | 5073 | 129 | NA | NA | NA | NA | 70 | NA | NA | NA | NA |
| | Mean ±SD | 116± 13 | 118± 17 | 111± 20 | 2± 3 | -11± 4# | 60± 6 | 61± 12 | 60± 12 | 0± 5 | -5± 4# |

Note: "*" p<0.05 compared with the baseline value, "**" p<0.01 compared with the baseline value. "#" change value compared with placebo group p<0.05, "##" change value compared with placebo group p<0.01.

### 5.5 Effect on heart function

See Table 17 for the effect on echocardiographic indicators in each group.

There was no obvious adverse reaction in the systolic and diastolic function in each dose group of the malate of the compound of formula I, Valsartan group and placebo group.

In addition, 2 cases of rhesus monkey animals (No. 5073 and No. 287) with low ejection fraction and cardiac insufficiency (SD) in the group with 5 mg/kg of the malate of compound of formula I for 8-week administration had increased LVEF% as compared with the baseline. However, whether this product can treat chronic heart failure needs to be supported by more follow-up research cohort data.

Table 17: Effect of the malate of compound of formula I on the main indexes of heart function in rhesus monkeys with spontaneous chronic kidney disease and evaluation of the therapeutic effect after 8-week administration

| Group | Animal No. | LVEF (%) | | Ea (cm/s) | | E/Ea | |
|---|---|---|---|---|---|---|---|
| | | Baseline | 8 weeks | Baseline | 8 weeks | Baseline | 8 weeks |
| Placebo group (n=4) | 6653 | 68.36 | 64.37 | 6.03 | 4.65 | 11.43 | 13.30 |
| | 231 | 54.05 | 59.26 | 5.10 | 4.94 | 10.87 | 11.59 |
| | 1029 | 50.79 | 50.71 | 4.02 | 5.16 | 10.63 | 10.13 |
| | 4713 | 58.81 | 55.63 | 4.26 | 4.60 | 26.16 | 22.38 |
| | Mean±SD | 58.00±7.65 | 57.49±5.77 | 4.85±0.91 | 4.84±0.26 | 14.77±7.60 | 14.35±5.51 |
| Valsartan group (n=3 ) | 2091 | 64.02 | 64.09 | 4.16 | 7.01 | 8.40 | 6.90 |
| | 4861 | 58.75 | 57.98 | 5.14 | 7.17 | 13.80 | 10.43 |
| | 6645 | 59.26 | 61.97 | 3.44 | 4.61 | 18.35 | 16.40 |
| | Mean±SD | 60.68±2.91 | 61.35±3.10 | 4.25±0.85 | 6.26±1.43 | 13.52±4.98 | 11.24±4.80 |
| The group with 5mg/kg of the malate of compound of Formula I (n=6) | 144 | 63.71 | 62.99 | 6.57 | 8.53 | 7.09 | 7.38 |
| | 5073 | 42.11 | 45.89 | 4.92 | 8.26 | 11.46 | 9.25 |
| | 6651 | 71.95 | 73.88 | 3.29 | 6.47 | 9.21 | 8.22 |
| | 257# | / | / | / | / | / | / |
| | 287 | 49.98 | 54.16 | 8.75 | 8.40 | 5.91 | 6.12 |
| | 4721 | 59.46 | 55.90 | 5.22 | 6.62 | 14.04 | 11.07 |
| | Mean±SD | 57.44±11.6 7 | 58.56±10.5 0 | 5.75±2.04 | 7.66±1.02 | 9.54±3.29 | 8.41±1.88 |
| The group with 2mg/kg of the malate of compound of Formula I (n=5 ) | 4829 | 54.41 | 58.54 | 4.36 | 5.00 | 17.11 | 10.63 |
| | 6501 | 70.41 | 67.80 | 6.37 | 6.66 | 7.73 | 6.35 |
| | 287 | 57.45 | 57.13 | 8.94 | 8.01 | 6.69 | 7.84 |
| | 4721 | 57.90 | 58.03 | 4.46 | 5.91 | 17.43 | 9.88 |
| | 2091 | 62.76 | 62.85 | 5.82 | 6.82 | 8.89 | 6.22 |
| | Mean±SD | 60.59±6.25 | 60.87±4.46 | 5.99±1.86 | 6.48±1.12 | 11.57±5.26 | 8.18±2.01 |

(continued)

| Group | Animal No. | LVEF (%) | | Ea (cm/s) | | E/Ea | |
|---|---|---|---|---|---|---|---|
| | | Baseline | 8 weeks | Baseline | 8 weeks | Baseline | 8 weeks |
| The group with 1mg/kg of the malate of compound of Formula I (n=5 ) | 1567 | 65.25 | 63.95 | 5.90 | 5.35 | 10.50 | 10.68 |
| | 4861 | 55.89 | 55.81 | 6.31 | 5.65 | 9.53 | 11.36 |
| | 5453 | 54.86 | 54.03 | 4.81 | 6.99 | 9.22 | 8.16 |
| | 6653 | 69.87 | 61.01 | 5.18 | 5.37 | 8.68 | 8.95 |
| | 5073 | 47.92 | 49.37 | 6.37 | 5.06 | 10.36 | 10.64 |
| | Mean±SD | 58.76±8.75 | 56.83±5.76 | 5.71±0.69 | 5.68±0.76 | 9.66±0.77 | 9.96±1.34 |

Remarks: The level of abnormal diastolic function was determined after comprehensive consideration of Ea, E/Ea, Ea/Aa and other functional indicators; when the abnormal level of diastolic function of the test animal was improved from the baseline, it was regarded as a "benefit". Otherwise, it was regarded as "invalid". For No. 257, the animal is with thoracic deformity, and cardiac ultrasound images cannot be collected.

### 5.6 Effect on Blood Biomarker of Heart Function Impairment

#### 5.6.1 Effect on NT-proBNP

The effect of the malate of the compound of formula I on NT-proBNP was shown in Table 18.
Compared with the baseline period, there was no significant change in NT-proBNP levels in each administration group after 8-week administration.

Table 18: Effect of the malate of compound of formula I on NT-proBNP in Rhesus Monkeys with Spontaneous Chronic Kidney Disease for 8-week administration

| Group | Animal No. | NT-proBNP (pg/ml) | | |
|---|---|---|---|---|
| | | Baseline | 4 weeks | 8 weeks |
| Placebo group (n=4) | 6653 | 342 | 143 | 172 |
| | 231 | 146 | 187 | 130 |
| | 1029 | 155 | 170 | 201 |
| | 4713 | 169 | 177 | 230 |
| | Mean±SD | 203±93 | 169±19 | 183±42 |
| Valsartan group (n=3) | 2091 | 211 | 179 | 217 |
| | 4861 | 500 | 260 | 288 |
| | 6645 | 394 | 285 | 291 |
| | Mean±SD | 368±146 | 241±56 | 266±42 |
| The group with 5mg/kg of the malate of compound of Formula I (n=6) | 144 | 312 | 254 | 261 |
| | 5073 | 88 | 108 | 146 |
| | 6651 | 211 | 245 | 250 |
| | 257 | 432 | 418 | 351 |
| | 287 | 177 | 210 | 197 |
| | 4721 | 162 | 178 | 180 |
| | Mean±SD | 230±123 | 236±104 | 231±73 |
| The group with 2mg/kg of the malate of compound of Formula I (n=5) | 4829 | 43 | 46 | 54 |
| | 6501 | 155 | 59 | 178 |
| | 287 | 335 | 304 | 253 |
| | 4721 | 46 | 46 | 54 |
| | 2091 | 250 | 107 | 174 |
| | Mean±SD | 166±127 | 113±110 | 143±87 |
| The group with 1mg/kg of the malate of compound of Formula I (n=5) | 1567 | 88 | 44 | 70 |
| | 4861 | 299 | 239 | 157 |
| | 5453 | 127 | 37 | 136 |
| | 6653 | 203 | 304 | 141 |
| | 5073 | 44 | 40 | 39 |
| | Mean±SD | 152±101 | 133±129 | 109±51 |

#### 5.6.2 Effect on $K^+$

The effect of the malate of compound of formula Ion $K^+$ was shown in Table 19.
Compared with the baseline period, there was no significant change in serum $K^+$ levels of animals in each administration group for 8-week administration.

Table 19: Effect of the malate of compound of formula I on $K^+$ in rhesus monkeys with spontaneous chronic kidney disease for 8-week administration

| Group | Animal No. | $K^+$ (mmol/L) | | | | | |
|---|---|---|---|---|---|---|---|
| | | Baseline 1 | Baseline 2 | 2 weeks | 4 weeks | 6 weeks | 8 weeks |
| Placebo group (n=4) | 6653 | 4.47 | 4.70 | 3.98 | 4.85 | 4.45 | 4.15 |
| | 231 | 4.79 | 5.37 | 5.78 | 4.76 | 5.02 | 5.32 |
| | 1029 | 4.43 | 3.92 | 3.68 | 3.50 | 4.12 | 3.68 |
| | 4713 | 4.95 | 4.38 | 3.64 | 4.01 | 3.98 | 3.96 |
| | Mean±SD | 4.66±0.25 | 4.59±0.61 | 4.27±1.02 | 4.28±0.64 | 4.39±0.46 | 4.28±0.72 |
| Valsartan group (n=3) | 2091 | 4.94 | 4.63 | 5.38 | 5.68 | 5.28 | 5.94 |
| | 4861 | 3.86 | 4.27 | 4.47 | 4.55 | 4.81 | 4.50 |
| | 6645 | 4.31 | 5.07 | 5.43 | 4.88 | 5.09 | 4.95 |
| | Mean±SD | 4.37±0.54 | 4.66±0.40 | 5.09±0.54 | 5.04±0.58 | 5.06±0.24 | 5.13±0.74 |
| The group with 5mg/kg of the malate of compound of Formula I (n=6) | 144 | 4.78 | 5.43 | 4.91 | 4.33 | 4.53 | 4.26 |
| | 5073 | 4.54 | 4.74 | 4.55 | 4.55 | 4.16 | 4.64 |
| | 6651 | 4.93 | 4.03 | 6.07 | 5.46 | 4.77 | 4.49 |
| | 257 | 5.41 | 5.20 | 5.37 | 4.50 | 4.76 | 5.28 |
| | 287 | 5.16 | 4.75 | 5.05 | 4.61 | 5.58 | 5.00 |
| | 4721 | 4.52 | 4.72 | 3.93 | 3.51 | 3.60 | 3.26 |
| | Mean±SD | 4.89±0.35 | 4.81±0.48 | 4.98±0.73 | 4.49±0.62 | 4.57±0.66 | 4.49±0.70 |
| The group with 2mg/kg of the malate of compound of Formula I (n=5) | 4829 | 4.53 | 4.69 | 4.02 | 4.74 | 5.48 | 4.50 |
| | 6501 | 4.42 | 5.80 | 4.83 | 4.76 | 5.72 | 4.36 |
| | 287 | 4.92 | 4.77 | 4.73 | 4.55 | 4.90 | 4.47 |
| | 4721 | 3.43 | 4.07 | 4.43 | 4.31 | 4.26 | 3.93 |
| | 2091 | 6.19 | 5.40 | 5.08 | 4.76 | 4.99 | 4.77 |
| | Mean±SD | 4.70±1.00 | 4.95±0.67 | 4.62±0.41 | 4.62±0.20 | 5.07±0.57 | 4.41±0.31 |
| The group with 1mg/kg of the malate of compound of Formula I (n=5 ) | 1567 | 3.93 | 4.50 | 4.17 | 4.30 | 4.67 | 4.29 |
| | 4861 | 4.30 | 4.51 | 4.14 | 3.94 | 4.99 | 4.17 |
| | 5453 | 4.15 | 4.40 | 4.15 | 4.26 | 4.08 | 4.15 |
| | 6653 | 4.74 | 4.78 | 4.95 | 5.23 | 5.57 | 4.49 |
| | 5073 | 4.98 | 4.79 | 5.04 | 4.79 | 5.30 | 4.65 |
| | Mean±SD | 4.42±0.43 | 4.60±0.18 | 4.49±0.46 | 4.50±0.51 | 4.92±0.58 | 4.35±0.22 |

**5.7 Safety tolerance study**

During the administration period, there were no adverse events related to administration in the groups administered with malate of the compound of formula I, and no significant changes were observed in liver function, renal function, food intake, body weight, hematological indicators, etc.

**6 Conclusion**

The proteinuria (UACR) and the glomerular filtration rate $eGFR_{creat-cys}$ can be significantly improved by administering the malate of compound of formula I in an amount of 2-5mg/kg qd for 8 weeks. The effect thereof was equivalent to the Valsartan group.

After 8-week administration of the malate of compound of formula I in an amount of 1-5 mg/kg qd, the activity of lowering blood pressure was shown. At the same time, there was no risk of hyperkalemia under the test conditions.

**Example 2: Evaluation of preclinical safety profile of the compound of Formula I**

**1. Materials and methods**

**1.1 Test substance**

The compound of Formula I was prepared and confirmed according to the method in CN103562191B. The 0.5% of sodium carboxymethyl cellulose (CMC-Na) was used as a vehicle. The compound of Formula I was stored at room temperature, protected from light and wetness. For cynomolgus monkey safty studies, the test substance was suspended in 0.5% CMC-Na at the relevant concentrations. The homogeneity, concentration and one week stability of prepared suspension was analyzed to ensure proper administration of the reported doses. The six month accelerated stability of active pharmaceutical ingredient (API) was tested by the inventor.

**1.2 Animal husbandry**

Healthy male and female cynomolgus monkeys aged 3-4 years were supplied by Hainan New Source Biotech Co. Ltd. (Hainan, China). The body weight range at the start of treatment was 3.2-5.8 kg for male and 2.5-3.7 kg for female. They were housed for a 36-day acclimation period before study. Once a day in the morning, animals eat and drink freely.

This nonclinical laboratory study has been carried out in compliance with the guidelines from the China Food and Drug Administration (CFDA). This experiment was conducted in facilities approved by the Association for Assessment and Accreditation of Laboratory Animal Care International (AAA LAC), and animals were maintained in accordance with the Guide for the Care and Use of Laboratory Animals (Nussberger et al., 2008).

**1.3 Doses and treatment schedule**

Cynomolgus monkeys (5/sex/group) were selected using a computerized randomization procedure based on body weight, and the compound of Formula I was administered to the cynomolgus monkeys by nasogastric feeding at dose levels of 0 (control), 20, 100, 450 mg/kg/day in a volume of 5 ml/kg. Individual dose volumes were adjusted weekly based on body weight of animals. For all the groups, 2/3 of the animals were randomly selected and euthanized on day 28. The remaining animals were euthanized after 28-day drug free period.

**1.4 Clinical observations**

Mortality and clinical signs were evaluated daily from the beginning of the quarantine period. Each animal was examined at least twice daily for any change in behavior, reaction to treatment or illness after administration.

Observations included, but were not limited to the following: changes in skin and fur; eyes and mucous membranes; respiratory, circulatory, autonomic, central nervous systems and behavior patterns. Rectal temperature was measured before administration (twice), at 1 and 24 h after first administration, and before d26 administration and at 1 and 24 h after d26 administration. Body weight was measured every week. Food consumption was estimated for monkey every day. Ophthalmoscopy was conducted for the monkey study using a portable slit lamp (YZ2) and a direct ophthalmoscope (GFJY-01B).

**1.5 Laboratory test**

For the monkey study, at quarantine period (twice, d01 and d02), day 14, day 28 and day 56, the following parameters were measured as shown in Table 20. (1) Hematological and coagulation index examination were performed using a Bayer ADVIA2120 (Germany) and a Sysmex CA-1500 (Japan). (2) The serum biochemical examination was assessed using a HITACHI 7080 automated analyzer (Japan) and an Easylyte PLUS electrolyte analyzer (MEDICA, America). (3) The 24-h pooled urine of each animal was collected by a tray under each cage. Urinalysis was carried out using Uritest-300 (China). (4) The 24-h pooled faeces of each monkey were collected by a tray under each cage. Fecal occult blood was detected. (5) ACCESS 2 Chemiluminescence immunoassay was used for myocardial troponin (TropI, non-GLP) test.

Table 20: Parameters evaluated in hematology, serum chemistry and urinanalysis.

| Hematology | Serum chemistry | Urinanalysis | feces |
|---|---|---|---|
| Erythrocyte count (RBC) | Alanine aminotransferase (ALT) | Glucose | Occult blood |
| Hemoglobin concentration (HB) | Aspartate aminotransferase (AST) | pH | |
| Hematocrit (HCT) | Alkaline phosphatase (ALP) | Specific gravity | |
| Mean corpuscular volume (MCV) | Glucose (GLU) | Protein | |
| Mean corpuscular hemoglobin (MCH) | Total protein (TP) | Bilirubin | |
| Mean corpuscular hemoglobin concentration (MCHC) | Albumin (ALB) | Ketone | |
| Platelet (PLT) | Urea nitrogen (BUN) | Occult blood | |
| Total leukocyte count (WBC) | Creatinine (CREA) | Nitrite | |
| Lymphocytes (LYMPH) | Triglyceride (TG) | Urobilinogen | |
| Monocytes (MONO) | Total cholesterol (CHOL) | | |
| Eosinophils (EOS) | Total bilirubin (TBIL) | | |
| Basophils (BASO) | Gamma-glutamyl transpeptidase (GGT) | | |
| Neutrophils (NEUT) | Calcium (Ca) | | |
| Reticulocyte count (RETIC) | Phosphorus (P) | | |
| Prothrombin time (PT) | Creatine phosphokinase (CPK) | | |
| Activated partial thromboplastin time (APTT) | $K^+$, $Na^+$, $Cl^-$ | | |
| | TropI | | |

**1.6 Routine ECG analysis for the monkeys study**

Routine ECG analysis was performed at each scheduled time (day 1 after administration, d01 and d02 after administration, 1 and 24 h after administration on day 1, before administration, and 1 and 24 h after administration on day 26 and day 56). P-wave, R-wave, T-wave, P-R intervals, Q-T intervals, QRS duration and heart rate were recorded by an ECG system using derivation DII (ECG-6951E, Shanghai, China).

**1.7 Necropsy and histopathology**

A complete gross necropsy was conducted on all animals by visual inspection at the end of the exposure period (day 28) and the recovery phase (day 56). The following selected organs were trimmed, weighed and evaluated in terms of absolute weight and as a percentage of final body weight or brain weight: brain, heart, kidney, liver, spleen, thymus, testes, epididymides, uterus, ovaries, adrenals and thyroid (including the parathyroid). The following tissues were preserved in 10% neutral buffered formalin: brain, pituitary gland, thyroid (including the parathyroid), trachea, heart, pancreas, spleen, adrenal glands, prostate, ovaries, uterus (including cervix uteri and oviduct), vagina, testes, epididymides, seminal vesicle, esophagus, duodenum, jejunum, ileum, cecum, colon, rectum, mesenteric lymph nodes, Peyer's patches, submaxillary lymph nodes, aorta, eyes, skeletal muscle, sciatic nerve, femur (including metaphysis), mammary gland, sternum, salivary glands, spinal cord, urinary bladder, lung (including the bronchi), liver, kidneys, stomach, bone marrow (sternal), thymus, sternum, gallbladder, any gross lesions or masses. The lung tissue was inflated with fixative at the time of necropsy. All the preserved tissues were paraffin embedded, sectioned, stained with hematoxylin and eosin (HE) and examined microscopically. Bone marrow cellular morphology examination was conducted with both smear (sternum) and paraffin-embedded sternum section.

**1.8 Toxicokinetics**

Blood samples were obtained on day1 and day26 at 0, 0.5, 1.5, 3, 5, 7 and 24 h after administration. On each occasion, approximately 1.0 mL of blood was drawn from the vein and collected into EDTA-$K_2$ anticoagulant tubes. The blood samples were put on ice and then centrifuged (3500 rpm, 5min) at 4 °C to obtain plasma samples, which were kept frozen at about -80 °C until analysis. Toxicokinetics determination of the compound of Formula I was conducted at the Institute of Materia Medica, Chinese Academy of Sciences (Shanghai, China).

### 1.9 Statistical analysis

For each sex, body weight, rectal temperature, ECG, organ weight, hematological parameters and serum biochemical data were analyzed with a statistical software SAS 9.3. Firstly homogeneity of variance was analyzed using Levene's test. If P > 0.05, the one-way ANOVA analysis was employed. If P < 0.05, the Kruskal-Wallis test was used. If the resulting ANOVA p-value was < 0.05, a comparison of each group using the Dunnett T test was performed. If the resulting Kruskal-Wallis test p-value was < 0.05, then the Dunnett T test was performed after data rank conversion.

## 2. Results

### 2.1 Clinical observations

Slight declining body weight gain was observed in high dose compared with controls (2.7 ± 0.4 versus 3.2 ± 0.2 for females, 4.1 ± 0.5 versus 4.6 ± 0.5 for males) with no statistical significance.

Treatment related early death occurred in one high dose female on day23, with clinical signs including hypoactivity, hunch back, low body temperature, diarrhea, mild intestinal tympanites and thymus atrophy. There were no apparent clinical signs of toxicity related to treatment observed in other animals.

### 2.2 Hematology and coagulation

Increased NEUT% and decreased LYMPH% were seen in all treated females and in high-dose males. The increases of Fbg in mid- and high-dose animals and APTT in high-dose females were also observed (Table 21).

Table 21: Hematology data for monkeys treated with the compound of Formula I for 4 weeks.

| Group | Time | WBC ($\times 10^9 \cdot L^{-1}$) | NEUT (%) | LYMPH (%) | APTT(sec) | Fbg($g \cdot L^{-1}$) |
|---|---|---|---|---|---|---|
| **Female** | | | | | | |
| 0 mg/kg | d0 | 12.1 ± 1.9 | 34.6 ±9.4 | 60.9 ± 8.8 | 16.2 ± 1.9 | 1.9 ± 0.4 |
| | d14 | 14.3 ± 4.6 | 38.8 ± 12.0 | 55.6 ± 11.3 | 16.4 ± 1.7 | 2.2 ± 1.0 |
| | d26 | 12.8 ± 2.7 | 36.7 ± 12.9 | 55.5 ± 11.8 | 19.1 ± 1.6 | 2.1 ± 1.1 |
| | d56 | 13.6 ± 0.3 | 36.7 ± 10.0 | 55.2 ± 13.4 | 18.0 ± 0.6 | 2.7 ± 1.6 |
| 20 mg/kg | d0 | 15.3 ± 3.6 | 42.5 ± 18.8 | 51.3 ± 18.0 | 16.7 ± 0.8 | 2.0 ± 0.3 |
| | d14 | 16.0 ± 3.9 | 52.5 ± 10.4 | 40.8 ± 11.3 | 15.9 ± 1.0 | 2.9 ± 0.9 |
| | d26 | 19.1 ± 4.0 | 61.4 ± 13.2** | 33.1 ± 12.8* | 18.3 ± 1.4 | 2.8 ± 0.9 |
| | d56 | 12.4 ± 1.6 | 38.1 ± 7.1 | 53.1 ± 7.4 | 16.4 ± 1.1 | 1.7 ± 0.5 |
| 100 mg/kg | d0 | 10.9 ± 2.9 | 50.5 ± 17.3 | 44.0 ± 16.5 | 15.0 ± 1.6 | 1.9 ± 0.4 |
| | d14 | 14.0 ± 4.0 | 59.5 ± 5.3* | 33.2 ±4.4* | 14.5 ± 0.9* | 2.5 ± 0.4 |
| | d26 | 15.9 ± 6.2 | 63.2 ± 6.4** | 30.0 ± 6.8** | 19.7 ± 2.2 | 3.5 ± 0.8 |
| | d56 | 9.9 ± 1.0 | 45.2 ± 2.3 | 48.7 ± 0.6 | 15.8 ± 1.5 | 2.1 ± 0.3 |
| 450 mg/kg | d0 | 14.4 ± 1.7 | 43.3 ± 12.3 | 51.8 ± 12.2 | 16.9 ± 1.4 | 1.8 ± 0.3 |
| | d14 | 16.9 ± 7.8 | 63.5 ± 15.2** | 30.1 ± 14.3** | 18.6 ± 0.8* | 3.9 ± 0.8* |
| | d26 | 18.1 ± 3.7 | 67.5 ± 9.2** | 26.9 ± 8.9** | 20.1 ± 3.1 | 4.2 ± 0.7** |
| | d56 | 14.0 ±4.9 | 42.3 ± 0.6 | 53.3 ± 0.3 | 18.0 ±3.5 | 1.4 ± 0.0 |
| **Male** | | | | | | |
| 0 mg/kg | d0 | 13.8 ± 4.2 | 48.3 ± 19.7 | 44.7 ± 19.6 | 17.2 ± 1.9 | 3.1 ± 1.7 |
| | d14 | 15.3 ± 5.6 | 49.7 ± 9.0 | 43.2 ±9.8 | 17.7 ± 1.6 | 3.0 ± 1.0 |
| | d26 | 15.3 ± 5.3 | 48.5 ± 8.4 | 45.8 ± 7.5 | 19.5 ± 2.2 | 2.0 ± 0.3 |
| | d56 | 12.1 ± 2.3 | 30.3 ± 12.3 | 62.1 ± 9.2 | 17.3 ± 1.2 | 1.8 ± 0.2 |
| 20 mg/kg | d0 | 13.4 ± 2.5 | 39.9 ± 14.6 | 55.3 ± 14.6 | 16.4 ± 1.1 | 2.1 ± 0.6 |
| | d14 | 17.7 ± 9.3 | 43.1 ± 20.4 | 51.1 ± 19.6 | 17.8 ± 2.7 | 3.2 ± 0.8 |
| | d26 | 13.8 ± 6.5 | 35.1 ± 12.4 | 59.5 ± 11.9 | 19.5 ± 2.4 | 2.2 ± 0.4 |
| | d56 | 20.1 ± 4.2 | 38.5 ± 12.6 | 53.3 ± 14.3 | 21.3 ± 4.0 | 3.1 ± 1.4 |
| 100 mg/kg | d0 | 12.6 ± 2.3 | 32.6 ± 18.2 | 61.5 ± 16.2 | 16.6 ± 1.8 | 2.2 ± 0.3 |
| | d14 | 15.6 ± 6.6 | 45.2 ± 12.2 | 48.0 ± 12.2 | 15.5 ± 0.5 | 2.8 ± 0.7 |

(continued)

| | | | | | | |
|---|---|---|---|---|---|---|
| **Male** | | | | | | |
| | d26 | 20.3 ± 2.9 | 60.8 ± 10.5 | 32.1 ±9.4 | 22.5 ±2.5 | 4.1 ± 1.2** |
| | d56 | 14.1 ± 0.2 | 42.5 ± 11.2 | 52.7 ± 9.0 | 17.7 ± 1.0 | 1.7 ± 0.3 |
| 450 mg/kg | d0 | 12.0 ± 3.8 | 46.9 ± 17.3 | 48.6 ± 16.8 | 17.0 ± 2.1 | 2.2 ± 0.7 |
| | d14 | 16.7 ± 6.4 | 59.2 ± 16.0 | 35.6 ± 15.5 | 16.1 ± 0.8 | 3.1 ± 1.0 |
| | d26 | 18.6 ± 5.9 | 68.6 ± 12.3* | 26.5 ± 12.0* | 20.0 ± 3.3 | 4.1 ± 0.6** |
| | d56 | 14.6 ± 0.8 | 36.7 ± 23.2 | 59.8 ± 22.8 | 19.9 ± 0.3 | 1.8 ± 0.0 |
| *p < 0.05, compared with control group, **p < 0.01, compared with control group Values are mean ± S.D. | | | | | | |

**2.3 Serum biochemistry**

Serum chemistry parameters CPK, BUN and CREA were significantly increased in high-dose cynomolgus monkeys of both sexes, and especially in the monkey that died early on d23 (d14 data, BUN: 34.99; CREA: 526). CHOL in high-dose males was significantly decreased. Serum Na level of high-dose males decreased slightly, but statistically significantly on d26. Other statistically significant findings were isolated occurrences and considered of no toxicological significance (Table 22).

Table 22: Serum chemistry for monkeys treated with the compound of Formula I for 4 weeks.

| **Group** | **Day** s | CPK ($U \cdot L^{-1}$) | BUN ($mmol \cdot L^{-1}$) | CREA ($\mu mol \cdot s^{-1} \cdot L^{-1}$) | CHOL ($mmol \cdot L^{-1}$) | Na ($mmol \cdot L^{-1}$) | K ($mmol \cdot L^{-1}$) |
|---|---|---|---|---|---|---|---|
| **Female** | | | | | | | |
| 0 mg/kg | d0 | 188.4 ± 83.4 | 6.2 ± 1.3 | 57.4 ± 4.9 | 3.3 ± 1.1 | 150.4 ± 2.3 | 4.7 ± 0.3 |
| | d14 | 180.0 ± 91.3 | 5.5 ± 1.0 | 57.8 ± 4.8 | 3.2 ± 1.2 | 149.3 ± 3.3 | 5.4 ± 0.4 |
| | d26 | 147.4 ± 42.2 | 5.6 ± 0.6 | 51.0 ± 5.5 | 3.1 ± 0.8 | 148.3 ± 1.8 | 5.0 ± 0.5 |
| | d56 | 135.5 ± 34.6 | 6.3 ± 0.6 | 49.0 ± 4.2 | 2.4 ± 0.4 | 147.5 ± 1.6 | 4.3 ± 0.1 |
| 20 mg/kg | d0 | 158.4 ± 63.5 | 6.1 ± 1.1 | 59.0 ± 4.0 | 3.0 ± 0.5 | 149.4 ± 3.5 | 5.0 ± 0.3 |
| | d14 | 151.6 ± 45.2 | 5.4 ± 0.9 | 52.4 ± 3.8 | 2.8 ± 0.2 | 149.6 ± 1.7 | 5.2 ± 0.2 |
| | d26 | 149.4 ± 58.7 | 6.3 ± 1.1 | 51.2 ± 3.6 | 2.7 ± 0.3 | 149.2 ± 4.3 | 4.9 ± 0.5 |
| | d56 | 129.0 ± 5.7 | 6.0 ± 0.6 | 51.5 ± 4.9 | 3.0 ± 0.1 | 147.2 ± 4.2 | 5.0 ± 0.5 |
| 100 mg/kg | d0 | 187.6 ± 75.0 | 5.9 ± 1.1 | 55.2 ± 4.7 | 3.8 ± 1.3 | 149.4 ± 1.9 | 4.5 ± 0.4 |
| | d14 | 247.2 ± 89.5 | 6.4 ± 0.8 | 55.2 ± 3.8 | 2.9 ± 0.6 | 149.8 ± 3.7 | 4.7 ± 0.5* |
| | d26 | 195.0 ± 96.8 | 6.4 ± 1.6 | 51.2 ± 3.6 | 3.1 ± 0.5 | 149.6 ± 3.8 | 4.8 ± 0.6 |
| | d56 | 112.0 ± 12.7 | 5.2 ± 0.2 | 54.5 ± 4.9 | 3.6 ± 0.2 | 147.9 ± 1.3 | 4.2 ± 0.2 |
| 450 mg/kg | d0 | 163.2 ± 56.2 | 6.1 ± 0.7 | 54.4 ± 7.1 | 3.3 ± 0.4 | 150.1 ± 2.2 | 5.1 ± 0.5 |
| | d14 | 815.2 ± 1316.0 | 14.3 ± 11.8 | 162.0 ± 204.0 | 2.4 ± 0.9 | 144.6 ± 3.5 | 5.5 ± 0.2 |

(continued)

| Group | Day s | CPK ($U \cdot L^{-1}$) | BUN ($mmol \cdot L^{-1}$) | CREA ($\mu mol \cdot s^{-1} \cdot L^{-1}$) | CHOL ($mmol \cdot L^{-1}$) | Na ($mmol \cdot L^{-1}$) | K ($mmol \cdot L^{-1}$) |
|---|---|---|---|---|---|---|---|
| **Female** | | | | | | | |
| | d26 | 328.5 ± 259.1 | 9.7 ± 1.0** | 64.3 ± 7.1 * | 2.0 ± 0.7 | 145.0 ± 2.6 | 5.3 ± 0.4 |
| | d56 | 290.0 ± 176.8 | 5.6 ± 0.6 | 57.0 ± 2.8 | 3.1 ± 0.3 | 145.7 ± 0.9 | 4.8 ± 0.2 |
| **Male** | | | | | | | |
| 0 mg/kg | d0 | 137.2 ± 56.1 | 5.6 ± 0.9 | 63.6 ± 4.3 | 3.1 ± 0.4 | 150.3 ± 3.2 | 4.9 ± 0.5 |
| | d14 | 152.0 ± 54.9 | 5.2 ± 1.0 | 59.4 ± 5.4 | 3.2 ± 0.3 | 150.5 ± 3.2 | 5.1 ± 0.3 |
| | d26 | 176.4 ± 91.1 | 5.7 ± 0.7 | 59.4 ± 3.0 | 2.9 ± 0.4 | 149.0 ± 2.0 | 4.7 ± 0.2 |
| | d56 | 343.5 ± 352.8 | 5.8 ± 0.1 | 60.0 ± 1.4 | 2.9 ± 0.2 | 147.3 ± 3.2 | 4.6 ± 0.0 |
| 20 mg/kg | d0 | 156.6 ± 76.3 | 5.1 ± 0.9 | 64.2 ± 7.2 | 2.9 ± 0.5 | 148.7 ± 2.0 | 5.1 ± 0.3 |
| | d14 | 128.2 ± 50.8 | 4.8 ± 0.5 | 65.0 ± 4.3 | 2.6 ± 0.4 | 148.7 ± 1.6 | 4.6 ± 0.3 |
| | d26 | 154.4 ± 21.5 | 5.6 ± 0.3 | 64.0 ± 5.5 | 2.7 ± 0.5 | 147.9 ± 1.9 | 5.4 ± 0.9 |
| | d56 | 161.0 ± 87.7 | 5.2 ± 0.4 | 64.0 ± 5.7 | 2.6 ± 0.1 | 144.6 ± 1.6 | 5.3 ± 1.1 |
| 100 mg/kg | d0 | 161.4 ± 12.3 | 5.7 ± 0.5 | 66.6 ± 8.6 | 3.6 ± 0.4 | 149.1 ± 2.9 | 4.8 ± 0.4 |
| | d14 | 213.4 ± 67.1 | 6.1 ± 1.5 | 63.0 ± 4.1 | 3.0 ± 0.6 | 148.3 ± 2.5 | 4.8 ± 0.4 |
| | d26 | 179.8 ± 42.1 | 5.9 ± 1.0 | 63.0 ± 8.5 | 2.8 ± 0.3 | 147.1 ± 2.4 | 5.2 ± 0.2 |
| | d56 | 109.0 ± 9.9 | 5.7 ± 1.5 | 58.5 ± 3.5 | 2.8 ± 0.6 | 146.5 ± 0.7 | 4.2 ± 0.0 |
| 450 mg/kg | d0 | 140.4 ± 44.6 | 5.3 ± 0.6 | 68.6 ± 5.1 | 3.4 ± 0.5 | 148.4 ± 2.5 | 5.2 ± 0.8 |
| | d14 | 290.2 ± 120.0* | 11.3 ± 6.7* | 115.8 ± 74.4 | 1.8 ± 0.3** | 146.4 ± 3.3 | 5.0 ± 0.2 |
| | d26 | 133.6 ± 44.6 | 10.4 ± 3.1** | 79.0 ± 2.8** | 1.7 ± 0.3** | 144.3 ± 2.2** | 5.2 ± 0.2 |
| | d56 | 99.5 ± 7.8 | 5.1 ± 0.0 | 66.5 ± 3.5 | 2.8 ± 0.3 | 145.3 ± 1.1 | 4.8 ± 0.8 |

*p < 0.05, compared with control group, **p < 0.01, compared with control group Values are mean ± S.D.

**2.4 Urinanalysis**

No significant changes were reported in any of the urinalysis parameters examined in either male or female monkeys.

### 2.5 ECG parameters

Prolonged QTc intervals were seen in one mid-dose female at 1h after administration on d26 (296 ms for animal No. 303). Sinus arrhythmia was observed in one high-dose male at 1h after administration on d26 with prolonged QRS intervals (60 ms for animal No. 411). Prolonged QRS intervals were also seen in high-dose females at 1h after administration on d1 and d26. (Table 23).

Table 23: ECG data for monkeys treated with the compound of Formula I for 4 weeks.

| Group | Days | HR (bmp) | QRS (ms) | QT (ms) | QTc (ms) |
|---|---|---|---|---|---|
| **Female** | | | | | |
| 0 mg/kg | d0 | 187.2 ± 39.6 | 32.0 ± 4.5 | 168.0 ± 19.2 | 243.1 ± 15.5 |
| | 1h(d1) | 244.4 ± 55.0 | 32.0 ± 4.5 | 154.0 ± 11.4 | 243.6 ± 12.1 |
| | 24h(d1) | 216.7 ± 18.3 | 32.0 ± 4.5 | 166.0 ± 15.2 | 254.4 ± 22.8 |
| | 1h(d26) | 259.2 ± 13.4 | 30.0 ± 0.0 | 142.0 ± 17.9 | 230.8 ± 25.7 |
| | 24h(d26 | 256.3 ± 10.7 | 30.0 ± 0.0 | 148.0 ± 8.4 | 240.0 ± 11.0 |
| | d56 | 222.8 ± 16.3 | 25.0 ± 7.1 | 170.0 ± 14.1 | 262.9 ± 15.5 |
| 20 mg/kg | d0 | 185.7 ± 48.0 | 34.0 ± 5.5 | 170.0 ± 20.0 | 244.6 ± 14.3 |
| | 1h(d1) | 230.1 ± 20.5 | 38.0 ± 4.5 | 160.0 ± 7.1 | 250.2 ± 10.4 |
| | 24h(d1) | 209.8 ± 9.3 | 34.0 ± 8.9 | 158.0 ± 4.5 | 239.7 ± 5.4 |
| | 1h(d26) | 262.8 ± 13.5 | 36.0 ± 5.5 | 140.0 ± 7.1 | 228.9 ± 8.0 |
| | 24h(d26 | 245.4 ± 43.9 | 36.0 ± 5.5 | 150.0 ± 10.0 | 238.6 ± 14.1 |
| | d56 | 238.0 ± 29.2 | 40.0 ± 0.0 | 160.0 ± 0.0 | 253.1 ± 10.4 |
| 100 mg/kg | d0 | 182.3 ± 28.9 | 32.0 ± 4.5 | 170.0 ± 12.2 | 245.0 ± 9.2 |
| | 1h(d1) | 228.5 ± 12.8 | 36.0 ± 5.5 | 160.0 ± 7.1 | 249.6 ± 6.7 |
| | 24h(d1) | 217.6 ± 22.7 | 34.0 ± 5.5 | 162.0 ± 11.0 | 248.2 ± 7.7 |
| | 1h(d26) | 246.4 ± 40.7 | 38.0 ± 4.5 | 170.0 ± 10.0* | 271.1 ± 17.2** |
| | 24h(d26 | 256.1 ± 17.1 | 38.0 ± 4.5 | 148.0 ± 8.4 | 239.8 ± 10.5 |
| | d56 | 230.8 ± 5.0 | 30.0 ± 0.0 | 160.0 ± 0.0 | 250.7 ± 1.8 |
| 450 mg/kg | d0 | 174.1 ± 28.8 | 32.0 ± 4.5 | 168.0 ± 16.4 | 238.1 ± 12.7 |
| | 1h(d1) | 211.3 ± 20.5 | 50.0 ± 14.1** | 175.0 ± 17.3 | 265.5 ± 19.9 |
| | 24h(d1) | 211.3 ± 2.4 | 35.0 ± 5.8 | 162.5 ± 9.6 | 247.2 ± 13.8 |
| | 1h(d26) | 262.7 ± 15.4 | 40.0 ± 0.2** | 140.0 ± 8.2 | 229.0 ± 14.6 |
| | 24h(d26 | 257.8 ± 9.0 | 32.5 ± 5.0 | 152.5 ± 9.6 | 247.8 ± 14.4 |
| | d56 | 207.4 ± 28.1 | 25.0 ± 7.1 | 170.0 ± 14.1 | 256.3 ± 9.7 |
| **Male** | | | | | |
| 0 mg/kg | d0 | 214.3 ± 12.6 | 36.0 ± 8.9 | 166.0 ± 8.9 | 253.7 ± 15.6 |
| | 1h(d1) | 248.4 ± 16.7 | 36.0 ± 5.5 | 152.0 ± 4.5 | 243.9 ± 6.6 |
| | 24h(d1) | 208.5 ± 18.5 | 34.0 ± 5.5 | 170.0 ± 14.1 | 256.9 ± 15.8 |
| | 1h(d26) | 253.9 ± 12.1 | 32.0 ± 4.5 | 148.0 ± 8.4 | 239.2 ± 10.5 |
| | 24h(d26 | 232.1 ± 43.2 | 38.0 ± 8.4 | 156.0 ± 8.9 | 243.3 ± 8.7 |
| | d56 | 194.5 ± 19.5 | 40.0 ± 0.0 | 180.0 ± 0.0 | 266.3 ± 8.9 |
| 20 mg/kg | d0 | 198.4 ± 16.5 | 34.0 ± 8.9 | 164.0 ± 11.4 | 243.9 ± 11.7 |
| | 1h(d1) | 216.0 ± 27.0 | 32.0 ± 4.5 | 158.0 ± 11.0 | 241.4 ± 12.3 |
| | 24h(d1) | 206.1 ± 20.7 | 28.0 ± 4.5 | 166.0 ± 8.9 | 250.0 ± 10.4 |
| | 1h(d26) | 260.0 ± 12.4 | 36.0 ± 5.5 | 146.0 ± 11.4 | 237.8 ± 16.3 |
| | 24h(d26 | 264.9 ± 16.1 | 34.0 ± 5.5 | 146.0 ± 11.4 | 239.2 ± 14.8 |
| | d56 | 225.6 ± 2.4 | 30.0 ± 0.0 | 145.0 ± 7.1 | 225.4 ± 10.2 |
| 100 mg/kg | d0 | 215.4 ± 11.2 | 32.0 ± 8.4 | 154.0 ± 16.7 | 235.6 ± 24.5 |
| | 1h(d1) | 230.0 ± 19.9 | 38.0 ± 4.5 | 156.0 ± 11.4 | 243.8 ± 16.5 |
| | 24h(d1) | 208.2 ± 10.4 | 36.0 ± 8.9 | 162.0 ± 11.0 | 245.3 ± 18.6 |
| | 1h(d26) | 256.9 ± 16.7 | 36.0 ± 5.5 | 144.0 ± 5.5 | 233.8 ± 12.2 |
| | 24h(d26 | 260.7 ± 9.1 | 36.0 ± 5.5 | 144.0 ± 8.9 | 234.9 ± 13.9 |

(continued)

| | | | | | |
|---|---|---|---|---|---|
| Male | | | | | |
| | d56 | 225.6 ± 2.4 | 35.0 ± 7.1 | 165.0 ± 7.1 | 256.6 ± 11.9 |
| 450 mg/kg | d0 | 214.3 ± 17.7 | 34.0 ± 5.5 | 156.0 ± 5.5 | 238.2 ± 7.2 |
| | 1h(d1) | 217.4 ± 3.2 | 36.7 ± 5.8 | 170.0 ± 10.0 | 261.1 ± 16.0 |
| | 24h(d1) | 211.0 ± 15.9 | 32.0 ± 4.5 | 166.0 ± 8.9 | 252.1 ± 9.4 |
| | 1h(d26) | 247.6 ± 22.6 | 36.0 ± 13.4 | 150.0 ± 17.3 | 240.0 ± 23.7 |
| | 24h(d26 | 210.4 ± 49.9 | 38.0 ± 4.5 | 158.0 ± 13.0 | 237.7 ± 12.2 |
| | d56 | 203.3 ± 15.5 | 35.0 ± 7.1 | 160.0 ± 0.0 | 240.2 ± 6.1 |

*p < 0.05, compared with control group, **p < 0.01, compared with control group. Values are mean ± S.D.

## 2.6 Necropsy

### 2.6.1 Gross pathology and organ weights

There were no macroscopic findings for monkeys deemed related to administration of the test article except mild intestinal tympanites and thymus atrophy for the monkey that died early. Higher relative weight of liver and kidney, lower relative weight of thymus and lower absolute or relative weight of uterus occurred in high-dose monkeys (Table 24).

Table 24: Organ weight for cynomolgus monkeys treated with the compound of Formula I for 4 weeks.

| Group | Time | Absolute weight(g) | | | |
|---|---|---|---|---|---|
| | | Liver | kidney | Thymus | Uterus |
| Female | | | | | |
| 0 mg/kg | d28 | 58.43±5.40 | 12.17±1.46 | 1.60±0.46 | 8.33±0.51 |
| | d56 | 69.80±6.51 | 14.55±0.64 | 1.95±2.05 | 6.05±2.76 |
| 20 mg/kg | d28 | 58.37±7.38 | 12.20±2.23 | 1.23±0.40 | 5.97±1.20 |
| | d56 | 68.35±3.32 | 13.75±0.78 | 2.00±0.71 | 11.30±3.25 |
| 100 mg/kg | d28 | 57.60±4.25 | 11.67±0.71 | 0.73±0.50 | 7.03±0.94 |
| | d56 | 68.45±20.01 | 14.15±2.33 | 2.35±0.49 | 8.30±3.39 |
| 450 mg/kg | d28 | 69.40±3.68 | 12.95±0.07 | 0.50±0.14 | 3.50±0.14** |
| | d56 | 72.40±11.74 | 14.75±1.91 | 1.40±0.14 | 9.60±2.40 |
| Male | | | | | |
| 0 mg/kg | d28 | 74.10±3.60 | 15.80±2.55 | 1.47±0.83 | |
| | d56 | 82.90±16.40 | 19.15±0.49 | 1.75±0.92 | |
| 20 mg/kg | d28 | 69.03±2.87 | 14.30±1.42 | 1.10±0.20 | |
| | d56 | 71.25±1.77 | 16.40±3.11 | 2.95±0.07 | |
| 100 mg/kg | d28 | 72.93±14.69 | 15.13±3.62 | 1.57±0.90 | |
| | d56 | 84.75±5.59 | 17.85±1.91 | 2.45±0.35 | |
| 450 mg/kg | d28 | 94.37±12.72 | 16.77±2.31 | 0.90±0.10 | |
| | d56 | 80.90±4.95 | 18.75±0.21 | 2.20±0.42 | |
| Female | | Relative weight (/body weight) | | | |
| 0 mg/kg | d28 | 1.83±0.22 | 0.38±0.023 | 0.05±0.01 | 0.26±0.03 |
| | d56 | 2.12±0.08 | 0.45±0.08 | 0.06±0.05 | 0.18±0.06 |
| 20 mg/kg | d28 | 2.06±0.22 | 0.43±0.07 | 0.04±0.01 | 0.21±0.04 |
| | d56 | 2.01±0.07 | 0.41+0.06 | 0.06±0.02 | 0.33±0.07 |
| 100 mg/kg | d28 | 1.96±0.13 | 0.40±0.02 | 0.02±0.02 | 0.24±0.03 |
| | d56 | 1.86±0.37 | 0.39±0.03 | 0.07±0.02 | 0.23±0.12 |
| 450 mg/kg | d28 | 2.78±0.31** | 0.52±0.03* | 0.02±0.01 | 0.14±0.01* |
| | d56 | 2.04±0.05 | 0.42±0.00 | 0.04±0.01 | 0.27±0.03 |

(continued)

| | | | | | |
|---|---|---|---|---|---|
| Male | | | | | |
| 0 mg/kg | d28 | 1.77±0.15 | 0.38±0.04 | 0.03±0.02 | |
| | d56 | 1.60±0.25 | 0.37±0.01 | 0.03±0.02 | |
| 20 mg/kg | d28 | 1.82±0.21 | 0.37±0.02 | 0.03±0.01 | |
| | d56 | 1.44±0.02 | 0.33±0.06 | 0.06±0.00 | |
| 100 mg/kg | d28 | 1.83±0.25 | 0.37±0.06 | 0.04±0.03 | |
| | d56 | 1.76±0.06 | 0.37±0.03 | 0.05±0.01 | |
| 450 mg/kg | d28 | 2.42±0.20* | 0.43±0.03 | 0.02±0.01 | |
| | d56 | 1.64+0.22 | 0.38±0.02 | 0.04±0.01 | |
| **Female** | | **Relative weight (/brain weight)** | | | |
| 0 mg/kg | d28 | 97.71±14.77 | 20.21±1.89 | 2.66±0.74 | 13.93±1.90 |
| | d56 | 108.79±9.78 | 22.68±1.07 | 3.03±3.19 | 9.42±4.27 |
| 20 mg/kg | d28 | 90.10±7.72 | 18.73±1.74 | 1.87±0.46 | 9.19±1.57 |
| | d56 | 104.00±10.62 | 20.86±0.06 | 3.07±1.24 | 17.30±5.86 |
| 100 mg/kg | d28 | 96.55±7.51 | 19.61±1.86 | 1.18±0.73 | 11.84±2.02 |
| | d56 | 104.90±37.06 | 21.60±4.92 | 3.54±0.52 | 12.44±4.34 |
| 450 mg/kg | d28 | 112.03±12.42 | 20.87±1.10 | 0.81±0.27* | 5.63±0.10** |
| | d56 | 109.03±13.64 | 22.23±2.05 | 2.12±0.29 | 14.43±3.09 |
| **Male** | | | | | |
| 0 mg/kg | d28 | 101.35±5.42 | 21.65±3.87 | 2.02±1.18 | |
| | d56 | 111.94±22.78 | 25.85±0.82 | 2.36±1.23 | |
| 20 mg/kg | d28 | 103.68±11.79 | 21.46±2.99 | 1.67±0.43 | |
| | d56 | 100.96±1.74 | 23.16±3.43 | 4.18±0.08 | |
| 100 mg/kg | d28 | 100.31±9.48 | 20.85±3.95 | 2.27±1.44 | |
| | d56 | 115.98±8.59 | 24.36±0.80 | 3.33±0.02 | |
| 450 mg/kg | d28 | 135.54±14.14* | 24.07±2.45 | 1.30±0.20 | |
| | d56 | 119.80±2.56 | 27.81±1.42 | 3.25±0.50 | |

*p < 0.05, compared with control group, **p < 0.01, compared with control group. Values are mean ± S.D.

2.7 Toxicokinetics

Systemic exposure, which was indicated by the plasma $AUC_{0-24h}$ and $C_{max}$ of parent compound and main metabolite, were proportionately greater than the magnitude of the dose increments in the dose range of 20 to 450 mg/kg. After consecutive 28 days of administration, the accumulation was not apparent either.

**Example 3: Nonclinical Pharmacokinetics**

**1. Materials and Methods**

**1.1 Materials**

The compound of Formula I was prepared and confirmed according to the method in CN103562191B.

**1.2 Animals**

Cynomolgus monkeys (3/sex/group: weight 3-5 kg, age 5-6 years old) were obtained from SuZhou XiShan ZhongKe Laboratory Animals (Shanghai, China). Animals were housed for a 7-day acclimation period before study. The monkeys were individually caged in a controlled temperature (18-26 °C) and humidity (50 ± 20%) animal room with an air flow of 10 plus air changes per hour with fresh air. The lighting cycle was 12 h on and 12 h off, and the monkeys were fed once a day in the morning. All animal experiments were performed in accordance with the Animal Care and Use Guidelines set by the AMMS Animal Care and Use Committee.

### 2. Intravenous and Oral Administration of the compound of Formula I

The compound of Formula I was intravenously or orally administered to cynomolgus monkeys fasted overnight. The doses were 1 mg/kg (intravenous), 1 mg/kg, 3 mg/kg, and 9 mg/kg (oral). Blood samples were collected from a limb vein at 0, 5, and 15 min and at 0.25, 0.5, 1, 2, 3, 4, 6, 8, 12, and 24 h (0.2 mL for each time point) after administration. All blood samples were centrifuged immediately after collection at 10,000×*g* for 5 min and the plasmas were stored at -75 ± 10 °C until analysis. Plasma samples were prepared by protein precipitation. The concentrations of the compound of Formula I in plasma were determined by the validated liquid chromatography-tandem mass spectrometry method.

The pharmacokinetic parameters such as the area under the plasma concentration-time curve (AUC), maximum plasma concentration ($C_{max}$), time to reach $C_{max}$ ($T_{max}$), half-life ($T_{1/2}$), total body plasma clearance (CL), mean residence time (MRT) and volume of distribution at steady state ($V_{SS}$) were calculated by non-compartmental analysis with WinNonlin v1.3 (Pharsight, Mountain View, CA, USA). For allometric scaling, animal pharmacokinetic data were simulated by a two-compartment model with the Kinetica 5.1 software package (v.3.0; InnaPhase, Philadelphia, PA, USA).

### 3. Results

Plasma Pharmacokinetics and bioavailability

Table 25 and Fig. 2 showed the pharmacokinetic data after intravenous and oral doses in cynomolgus monkeys. Drug concentration declined biexponentially after intravenous administration witha clearance of 1.96 L/h/kg and distribution volume of 1.86 L/kg for monkeys. After oral administration, the compound of Formula I reached a $C_{max}$at 1.0-1.30 h, and disappeared with a $T_{1/2}$ of 2.73-5.99 h in monkey plasma. The oral bioavailability of the compound of Formula I was about 3.3-11.3% in monkeys calculated from the $AUC_{0-24h}$ after iv and poadministration, which indicated that efflux transporters were involved.

Table 25

| Species | Dose mg/kg | Route | CL L/h/kg | Vss L/kg | MRT h | $t_{1/2}$ h | $C_{max}$ ng/ml | $T_{max}$ h | $AUC_{tot,}$ ng*h/ml | CL/F L/h/kg | Vss/F L/kg | F (%) |
|---|---|---|---|---|---|---|---|---|---|---|---|---|
| Monkey | 1 | *iv* | 1.96 | 1.86 | 0.97 | 2.73 | 466 | - | 421 | - | - | - |
| | 1 | *po* | - | - | 4.32 | 2.90 | 3.99 | 1.0 | 11.8 | 84.8 | 366 | 3.3 |
| | 3 | *po* | - | - | 3.46 | 3.68 | 42.2 | 1.3 | 99.4 | 30.2 | 104 | 8.0 |
| | 9 | *po* | - | - | 2.62 | 5.99 | 263 | 1.0 | 426 | 21.2 | 55.5 | 11.3 |

*CL* clearance after iv, $V_{ss}$ volume of distribution at steady state after iv, *MRT* mean residence time, $T_{½}$ half-life, $C_{max}$ maximum plasma concentration, $T_{max}$ time to reach $C_{max}$, $AUC_{tot}$ the total area under the plasma concentration-time curve, *CL/F* clearance after po, $V_{ss}/F$ volume of distribution at steady state after po, *F* absolute oral bioavailability, *iv* intrravenous, *po* oral.

## Claims

1. Use of a nitrogen-containing saturated heterocyclic compound represented by the following formula I or a pharmaceutically acceptable salt thereof in the preparation of a medicine for the treatment and/or prevention of chronic kidney disease:

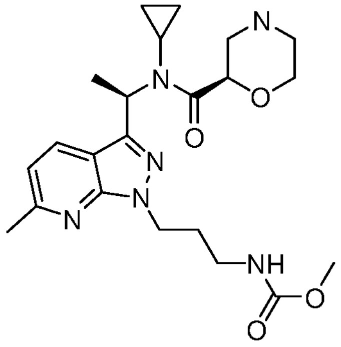

formula I .

2. The use according to claim 1, wherein, the pharmaceutically acceptable salt is hydrochloride, sulfate, phosphate, hydrobromide, acetate, fumarate, oxalate, citrate, methanesulfonate, benzenesulfonate, p-toluenesulfonate, maleate, malate, tartrate, acetate or naphthalene disulfonate.

3. The use according to claim 1 or 2, wherein, the pharmaceutically acceptable salt is the malate of the compound of formula I, which is a compound formed by the compound of formula I and malic acid at a molar ratio of 1:1, and the structural formula thereof is as follows:

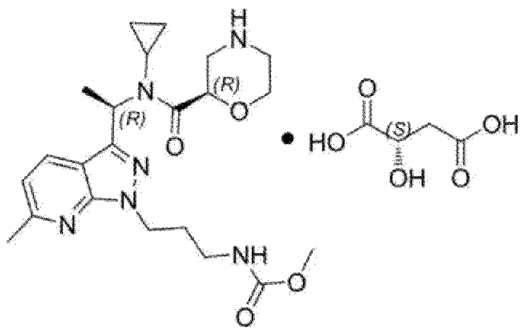

.

4. The use according to claim 3, wherein, the malate is a crystal form, the X-ray powder diffraction pattern thereof has characteristic peaks at 2θ of 7.767°±0.2°, 13.897°±0.2°, 14.775°±0.2°, 17.098°±0.2°, 18.999°±0.2°, 20.153±0.2°, 20.960°±0.2°, 21.423°±0.2°, 26.348°±0.2° and 27.892°±0.2°,
in particular, the X-ray powder diffraction pattern of the malate crystal further has characteristic peaks at 2θ of 5.598°±0.2°, 7.357°±0.2°, 10.395°±0.2°, 11.108°±0.2°, 16.037°±0.2°, 16.523°±0.2°, 19.410°±0.2°, 22.645°±0.2°, 26.630°±0.2°, 26.891°±0.2°, 27.380°±0.2°, 31.056° ±0.2°, 33.396°±9.2°, 33.775°±0.2° and 39.231°±0.2°.

5. The use according to claim 4, wherein, the malate crystal of the compound of formula I has an X-ray powder diffraction pattern as shown in Fig. 1.

6. The use according to any one of claims 1-5, wherein, the chronic kidney disease is hypertension with nephropathy, hypertension with nephropathy with abnormal glucose metabolism, chronic renal insufficiency with chronic heart failure or chronic kidney disease with abnormal glucose metabolism.

7. The use according to claim 6, wherein, the hypertension in hypertension with nephropathy is grade 1 hypertension, grade 2 hypertension or grade 3 hypertension, preferably grade 1 hypertension or grade 2 hypertension.

8. The use according to any one of claims 1-7, wherein, the chronic kidney disease refers to the G1, G2, G3a, G3b, G4 stages of chronic kidney disease, preferably the G2, G3a or G3b stage.

9. The use according to any one of claims 1-8, wherein, the dosage forms of the medicine include tablet, capsule, intravenous injection, inhalant, nebulizer, lyophilized agent, patch, gel, spray, or suppository, preferably tablet.

10. The use according to any one of claims 1-9, wherein, the medicine is unit dose, preferably, the unit dose contains the nitrogen-containing saturated heterocyclic compound represented by formula I or the pharmaceutically acceptable salt thereof in a range of 25 mg to 200 mg, for example, 25 mg, 50 mg, 100 mg, 150 mg, 200 mg of the nitrogen-containing saturated heterocyclic compound represented by formula I or the pharmaceutically acceptable salt thereof.

Fig. 1

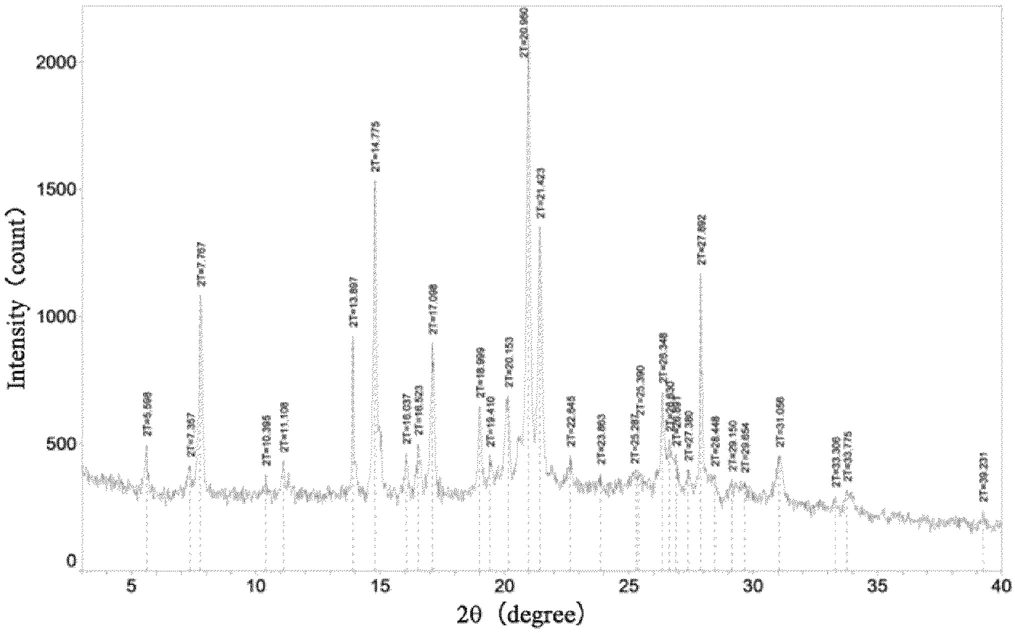
Intensity (count)
2θ (degree)

Fig. 2

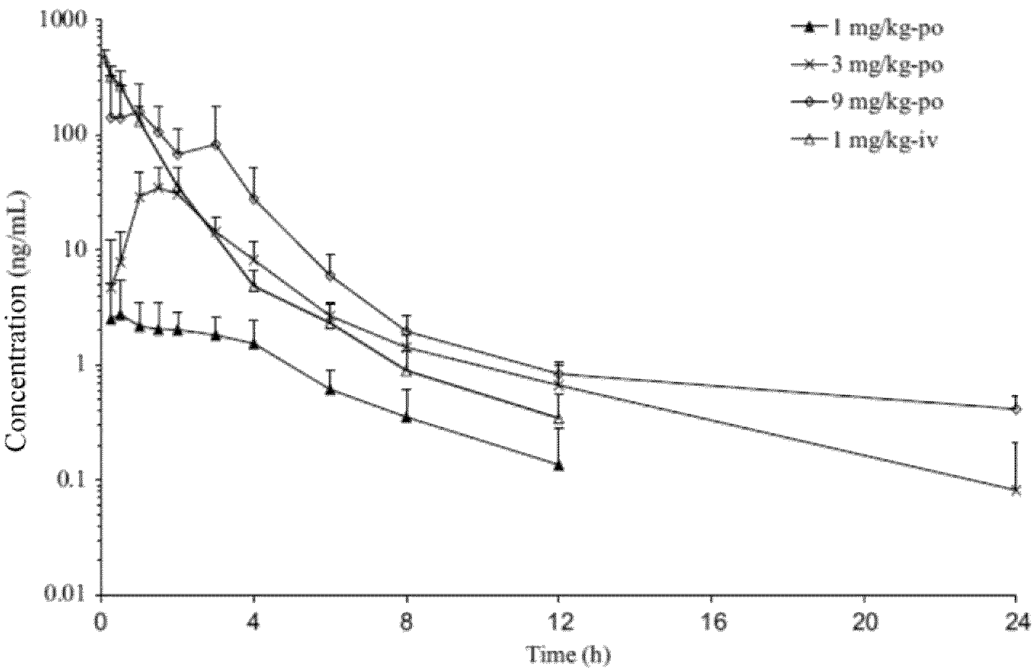
1 mg/kg-po
3 mg/kg-po
9 mg/kg-po
1 mg/kg-iv
Concentration (ng/mL)
Time (h)

## INTERNATIONAL SEARCH REPORT

International application No.
**PCT/CN2021/116338**

**A. CLASSIFICATION OF SUBJECT MATTER**

A61K 31/5377(2006.01)i; A61P 13/12(2006.01)i; A61P 9/12(2006.01)i; A61P 3/10(2006.01)i; A61P 9/04(2006.01)i; A61K 9/20(2006.01)i; A61K 9/48(2006.01)i; A61K 9/08(2006.01)i; A61K 9/19(2006.01)i; A61K 9/06(2006.01)i; A61K 9/02(2006.01)i; A61K 9/12(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

STN, 超星读秀学术: structural formula search, kidney, hypertension, diabetes, glucose metabolism, 肾素, 抑制, 慢性肾病, 血糖, 高血压, 糖尿病

**C. DOCUMENTS CONSIDERED TO BE RELEVANT**

| Category* | Citation of document, with indication, where appropriate, of the relevant passages | Relevant to claim No. |
|---|---|---|
| X | CN 106928218 A (SHANGHAI PHARMACEUTICALS HOLDING CO., LTD.; MITSUBISHI TANABE PHARMA CORPORATION) 07 July 2017 (2017-07-07) see claims 1-39, description pages 1-8, description figure 1 - figure 31 | 1-5, 9, 10 |
| X | CN 103562191 A (SHANGHAI PHARMACEUTICALS HOLDING CO., LTD.; MITSUBISHI TANABE PHARMA CORPORATION) 05 February 2014 (2014-02-05) see description abstract, description paragraphs 407-419, table 65 compound 270 | 1, 2, 9, 10 |
| X | JP 2014074024 A (MITSUBISHI TANABE PHARMA CORP.) 24 April 2014 (2014-04-24) see description abstract, description pages 25-28, table 65 compound 270 | 1, 2, 9, 10 |
| Y | CN 106928218 A (SHANGHAI PHARMACEUTICALS HOLDING CO., LTD.; MITSUBISHI TANABE PHARMA CORPORATION) 07 July 2017 (2017-07-07) see claims 1-39, description pages 1-8, description figure 1 - figure 31 | 6-8 |
| Y | CN 103562191 A (SHANGHAI PHARMACEUTICALS HOLDING CO., LTD.; MITSUBISHI TANABE PHARMA CORPORATION) 05 February 2014 (2014-02-05) see description abstract, description paragraphs 407-419, table 65 compound 270 | 6-8 |
| Y | JP 2014074024 A (MITSUBISHI TANABE PHARMA CORP.) 24 April 2014 (2014-04-24) see description abstract, description pages 25-28, table 65 compound 270 | 6-8 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 November 2021** | **07 December 2021** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/116338**

**C. DOCUMENTS CONSIDERED TO BE RELEVANT**

| Category* | Citation of document, with indication, where appropriate, of the relevant passages | Relevant to claim No. |
|---|---|---|
| Y | 李学军等主编 (Edited by LEE, Xuejun et al.). *《药理学》("Pharmacology")*, 30 November 2012 (2012-11-30), see page 248 paragraph 3, page 251 | 6-8 |

Form PCT/ISA/210 (second sheet) (January 2015)

**INTERNATIONAL SEARCH REPORT**
**Information on patent family members**

International application No.
**PCT/CN2021/116338**

| Patent document cited in search report | | | Publication date (day/month/year) | Patent family member(s) | | | Publication date (day/month/year) |
|---|---|---|---|---|---|---|---|
| CN | 106928218 | A | 07 July 2017 | EP | 3398946 | A1 | 07 November 2018 |
| | | | | EP | 3398946 | A4 | 28 August 2019 |
| | | | | WO | 2017114456 | A1 | 06 July 2017 |
| | | | | JP | 2019500385 | A | 10 January 2019 |
| | | | | JP | 6818031 | B2 | 20 January 2021 |
| | | | | TW | 201722950 | A | 01 July 2017 |
| | | | | TW | I705065 | B | 21 September 2020 |
| | | | | US | 2019016718 | A1 | 17 January 2019 |
| | | | | US | 10519150 | B2 | 31 December 2019 |
| CN | 103562191 | A | 05 February 2014 | CA | 2828378 | A1 | 20 September 2012 |
| | | | | CA | 2828378 | C | 14 November 2017 |
| | | | | CN | 103562191 | B | 30 March 2016 |
| | | | | KR | 20130133294 | A | 06 December 2013 |
| | | | | KR | 101651722 | B1 | 26 August 2016 |
| | | | | HU | E037584 | T2 | 28 September 2018 |
| | | | | PL | 2687518 | T3 | 30 April 2018 |
| | | | | US | 2014011807 | A1 | 09 January 2014 |
| | | | | US | 9278944 | B2 | 08 March 2016 |
| | | | | ES | 2652454 | T3 | 02 February 2018 |
| | | | | AU | 2012229859 | A1 | 19 September 2013 |
| | | | | AU | 2012229859 | B2 | 16 July 2015 |
| | | | | AU | 2012229859 | C1 | 16 January 2020 |
| | | | | EP | 2687518 | A1 | 22 January 2014 |
| | | | | EP | 2687518 | A4 | 20 August 2014 |
| | | | | EP | 2687518 | B1 | 01 November 2017 |
| | | | | WO | 2012124775 | A1 | 20 September 2012 |
| | | | | DK | 2687518 | T3 | 29 January 2018 |
| | | | | PT | 2687518 | T | 19 January 2018 |
| | | | | MX | 2013010554 | A | 02 December 2013 |
| | | | | MX | 349484 | B | 31 July 2017 |
| | | | | US | 2016145220 | A1 | 26 May 2016 |
| | | | | US | 10155731 | B2 | 18 December 2018 |
| | | | | BR | 112013023480 | A2 | 18 July 2017 |
| | | | | BR | 112013023480 | A8 | 09 July 2019 |
| | | | | BR | 112013023480 | B1 | 01 October 2019 |
| | | | | RU | 2013146012 | A | 27 May 2015 |
| | | | | RU | 2595136 | C2 | 20 August 2016 |
| | | | | TW | 201242955 | A | 01 November 2012 |
| | | | | TW | I597273 | B | 01 September 2017 |
| | | | | JP | 5760078 | B2 | 05 August 2015 |
| | | | | WO | 2012124775 | A1 | 24 July 2014 |
| JP | 2014074024 | A | 24 April 2014 | JP | 5764628 | B2 | 19 August 2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103562191 B
- CN 106928218 A